# EUROPEAN PATENT APPLICATION

(11) **EP 1 616 596 A2**
(43) Date of publication of application: **18.01.2006**
(21) Application number: 05109312.8
(22) Date of filing: 16.12.2003
(51) Int. Cl.: A61N 1/05, A61N 1/34

(54) **Apparatuses and methods for coupling percutaneous devices to a patient, and associated methods of manufacture**

(30) Priority: 16.12.2002 US 433868 P; 16.12.2002 US 433876 P
(62) Divisional of application: 03797021.7
(71) Applicant: MEAGAN MEDICAL, INC., Reno, Nevada 89509 (US)
(72) Inventor: Miazaga, Jay, N. Seattle, WA 98103, (US); Leonard, Paul C., Woodinville, WA 98072 (US); Genau, Chris, N. Seattle, WA 98103 (US)
(74) Representative: Edlund, Fabian

(57) **Abstract**

An apparatus for supporting couplers for removable coupling to a recipient during at least one of therapy administration and recipient monitoring. The apparatus comprises a support member configured to rest on a body of the recipient, the support member having a first coupler support portion configured to be positioned proximate to a first coupling position of the body of the recipient, and a second coupler support portion configured to be positioned proximate to a second coupling position of the body of the recipient. The first coupler support portion is configured to removably carry a first coupler, and the second coupler support portion is configured to removably carry a second coupler. The support member includes a deformable member positioned at least proximate to the first and second coupler support portions, the deformable member being bendable from a first shape to a second shape and configured to at least generally maintain its shape after being bent.

## Description

### REFERENCE TO RELATED APPLICATIONS

This application claims priority to the co-pending U.S. provisional application serial numbers 60/433,868, filed December 16, 2002, and 60/433,876, filed December 16, 2002, which are entirely incorporated herein by reference.

### TECHNICAL FIELD

This invention relates generally to apparatuses and methods for coupling therapeutic and/or monitoring equipment to a patient, and associated methods of manufacture.

### BACKGROUND

Electrical therapy has long been used in medicine to treat pain and other conditions. For example, transcutaneous electrical nerve stimulation (TENS) systems deliver electrical energy through electrode patches placed on the surface of a patient's skin to treat pain in tissue beneath and around the location of the patches. However, the TENS systems may not adequately alleviate pain in certain circumstances.

More recently, a technique in which electrodes are placed through the patient's skin into the target tissue has been proposed. Percutaneous Neuromodulation Therapy ("PNT") (also sometimes called Percutaneous Electrical Nerve Stimulation or "PENS") using percutaneously placed electrodes achieves significantly better pain relief results than TENS treatments using skin surface electrodes. That therapy is described in Ghoname *et al*., "Percutaneous Electrical Nerve Stimulation for Low Back Pain," JAMA 281:818-23 (1999); Ghoname *et al*., "The Effect of Stimulus Frequency on the Analgesic Response to Percutaneous Electrical Nerve Stimulation in Patients with Chronic Low Back Pain," Anesth. Analg. 88:841-6 (1999); Ahmed *et al*., "Percutaneous Electrical Nerve Stimulation (PENS): A Complementary Therapy for the Management of Pain Secondary to Bony Metastasis," Clinical Journal of Pain 14:320-3 (1998); and Ahmod *et al*., "Percutaneous Electrical Nerve Stimulation: An Alternative to Antiviral Drugs for Herpes Zoster," Anesth. Analg. 87:911-4 (1998). The contents of those references are incorporated herein by reference.

Thus far, PNT practitioners have used percutaneously placed acupuncture needles attached to waveform generators via cables and alligator clips to deliver the therapy to the patient. One feature of conventional PNT systems is that they typically include a number of electrical cables that must be properly connected to the corresponding percutaneous electrodes to deliver effective electrical therapy. Accordingly, a drawback with those conventional systems is that it can be difficult (particularly for inexperienced practitioners) to connect each electrical cable to the proper corresponding electrode. This drawback is shared as well by other systems that require multiple connections to the patient. Such systems include electrical monitoring systems and drug delivery systems.

Another feature of some existing medical devices is that they include electrical conductors which must be insulated and in some cases provided with a soft covering for patient comfort. Molding thermoplastic elastomers and other materials over wires and other semi-rigid members is a known method for providing a protective and/or flexible covering over such members. Molding elastomers over flexible members, however, is more difficult because the flexible members tend to stretch, flex or otherwise move within the mold as the uncured elastomer is injected into the mold. This movement can result in damage or "surfacing" of the flexible members. In the case of electrical wiring, surfacing can expose the wires to abrasion during use which can ultimately result in failure of the wires. Those problems tend to increase as the size of the molded part increases.

To overcome those problems, such parts are generally kept relatively small or, alternatively, the flexible members are externally stiffened to prevent deformation during the overmolding process. If neither of those approaches is possible, then features (often pins or ribs) can be provided in the mold tooling to capture or otherwise restrain the flexible member during the molding process. One disadvantage of that approach is that the features built into the mold tooling to capture the flexible member often leave holes or other irregularities in the exterior surface of the moldod part that have to be covered in a subsequent operation. If those holes are not covered, they may collect dirt and other contaminants - an undesirable characteristic, especially if the part is intended for medical applications where cleanliness is important.

### SUMMARY

The invention is directed to apparatuses and methods for supporting therapeutic and/or diagnostic couplers for removable coupling to a recipient. An apparatus in accordance with one aspect of the invention can include a U-shaped support member configured to rest on a body of the recipient proximate to a coupling region. The U-shaped support member can include a first coupler support portion positioned on a first leg of the U-shaped support member and a second coupler support portion positioned on a second leg of the U-shaped support member. The first coupler support portion can be configured to removably carry a first coupler proximate to a first coupling position of the body of the recipient, and the second coupler support portion can be configured to removably carry a second coupler proximate to a second coupling position of the body of the recipient. In one aspect of the invention, the first coupler support portion can be positioned closer than the second coupler support portion to the first coupling position. Accordingly, the apparatus can guide a practitioner to connect the couplers to the correct coupling position.

A method for manufacturing a flexible carrier in accordance with one aspect of the invention can include forming a first portion of the flexible carrier from a first quantity of elastic material. The first portion of the flexible carrier can have s channel, and at least a portion of a flexible member can be positioned in the channel. The method can further include forming a second portion of the flexible carrier by disposing an at least partially uncured second quantity of elastic material in the channel to at least partially cover the portion of the flexible member positioned in the channel.

In one aspect of the invention, forming the first portion of the flexible carrier can include filling a first mold assembly with the first quantity of elastic material when the first quantity of elastic material is at least partially uncured. After the first quantity of elastic material has at least partially cured, the first portion of the flexible carrier can be removed from the first mold assembly and positioned in a second mold assembly to receive the at least partially uncured second quantity of elastic material in the channel. The second quantity of elastic material can at least partially cover the portion of the flexible member positioned in the channel.

In another aspect of the invention, the method can further include positioning at least a portion of a deformable member in the flexible carrier. The deformable member can be bendable from a first shape to a second shape and configured to at least generally maintain its shape after being bent. In a further aspect of the invention, the deformable member can be positioned in the channel of the first portion of the flexible carrier.

In yet another aspect of the invention, the support member can be flexible and resilient to conform to a surface of the body, and can be shaped to rest on at least one of a back, a neck, a head, and a leg of the recipient. The apparatus can further include a flexible link coupled between the first coupler and the support member. The link can remain connected between the first coupler and the support member when the first coupler is moved from an attached position to a coupled position with the coupler coupled to the body of the recipient. The link can include an electrical cable configured to be coupled to a source of electrical pulses, an electrical cable configured to be coupled to a signal monitor, and/or a length of tubing configured to be coupled to a source of liquid medicament.

In an aspect of the invention, the first and second coupling positions can be two of a larger plurality of coupling positions and the first and second coupler location s can be two of a larger plurality of coupler locations. An outline of the coupling positions can define a fist shape and an outline of the coupler locations can define a corresponding second shape at least generally similar to the fist shape.

A method for coupling therapy and/or monitoring equipment to a recipient in accordance with one aspect of the invention can include bending at least a portion of a support member from a first shape to a second shape, the second shape at least generally conforming the support member to a curved surface of a body of the recipient. While the support member is bent into the second shape, the support member can be positioned against the curved surface of the body of the recipient proximate to a coupling area of the body and spaced apart from first and second coupling positions in the coupling area. The method can further include removing a first coupler from the support member and coupling the first coupler to the body at the first coupling position, and removing the second coupler from the support member and coupling the second coupler to the body at the second coupling position.

Another method for coupling therapy and/or monitoring equipment to a recipient in accordance with another aspect of the invention can include positioning first and second legs of a U-shaped support member against a body of a recipient proximate to first and second coupling positions in a coupling area of the body. The method can further include removing a first coupler from a first coupler support portion of the first leg of the support member and coupling the first coupler to the body at the first coupling position, and removing a second coupler from a second coupler support portion of the second leg of the support member and coupling the second coupler to the body at the second coupling position. In one aspect of the invention, the first coupler support portion can be positioned closer than the second coupler support portion to the first coupling position.

### BRIEF DESCRIPTION OF THE DRAWINGS

Figures 1A-G are schematic renderings of a percutaneous electrical therapy system according to one embodiment of the invention.

Figure 1A shows electrode and angle of insertion assemblies wherein the electrode is in an undeployed and uninserted state.

Figure 1B shows the electrode and angle of insertion assemblies of Figure 1A during deployment but prior to insertion of the electrode into a patient's tissue.

Figure 1C shows the electrode and angle of insertion assemblies of Figure 1A during deployment and insertion of the electrode into the patient's tissue.

Figure 1D shows the electrode of Figure 1A inserted into the patient's tissue.

Figure 1E shows the electrode of Figure 1A attached to a control unit to provide percutaneous electrical therapy.

Figure 1F shows the electrode and angle of insertion assemblies of Figure 1A during undeployment but prior to removing the electrode from the patient's tissue.

Figure 1G shows the electrode and sharp point protection assemblies of Figure 1A during undeployment and after removing the electrode from the patient's tissue.

Figures 2A-E are schematic renderings of a percutaneous electrical therapy system according to another embodiment of the invention.

Figure 2A shows a percutaneous electrical therapy system with electrode and angle of insertion assemblies wherein the electrode is in an undeployed and uninserted state.

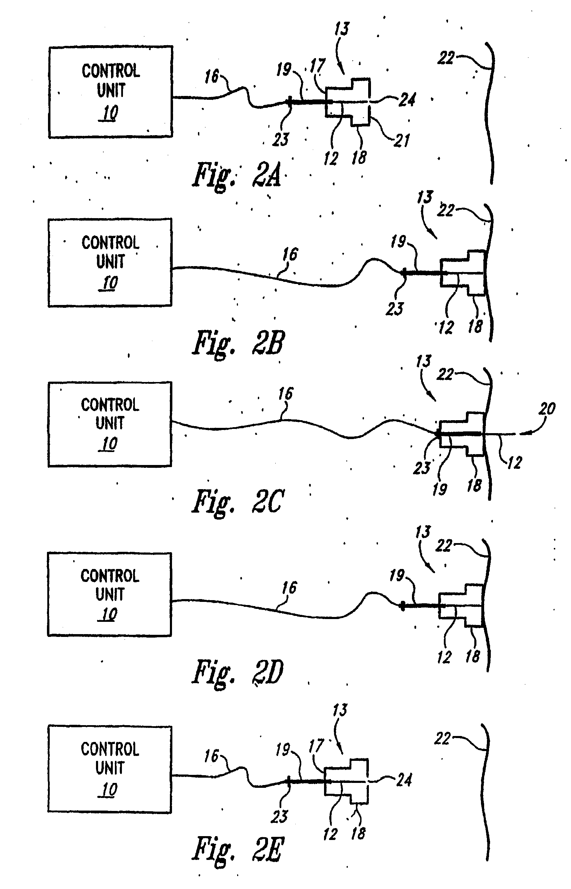

Figure 2B shows the percutaneous electrical therapy system of Figure 2A during deployment, but prior to insertion, of the electrode.

Figure 2C shows the percutaneous electrical therapy system of Figure 2A with the electrode in a deployed and inserted state.

Figure 2D shows the percutaneous electrical therapy system of Figure 2A during undeployment of the electrode.

Figure 2E shows the percutaneous electrical therapy system of Figure 2A after the electrode has been undeployed.

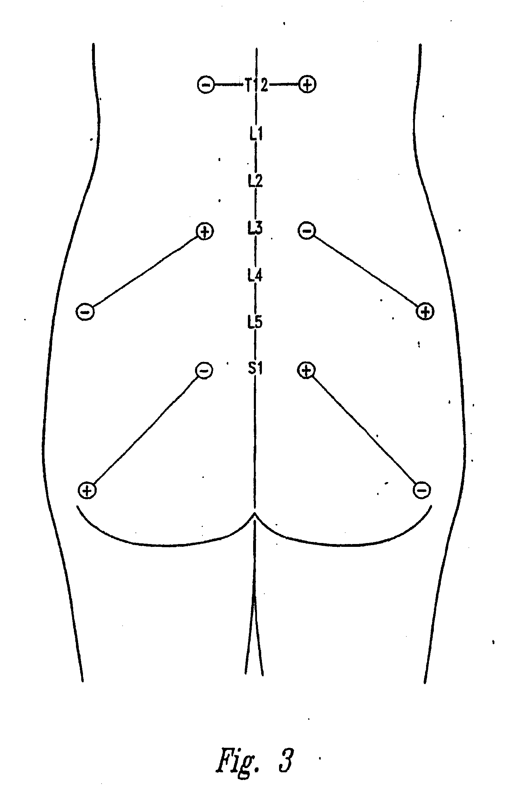

Figure 3 shows an electrode montage for use in percutaneous neuromodulation therapy to treat low back pain.

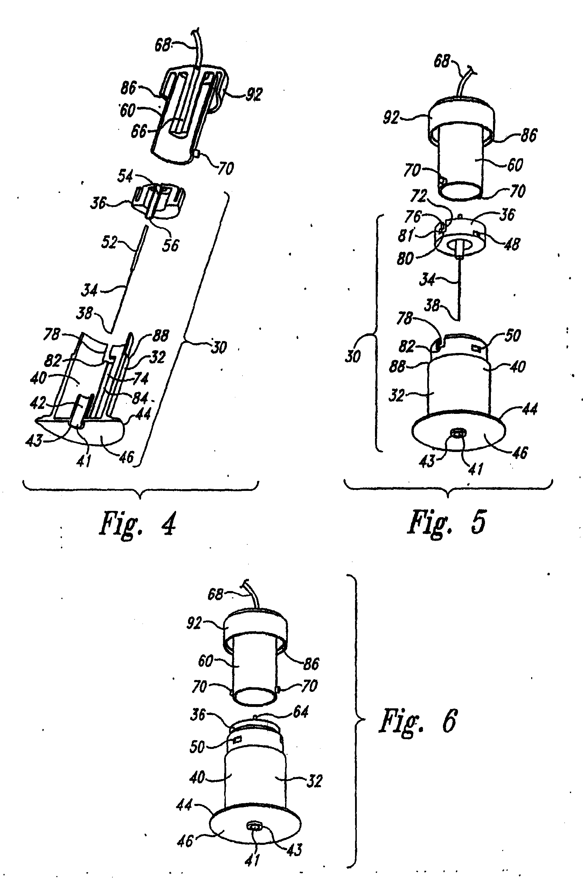

Figure 4 is an exploded sectional view of an electrode and angle of insertion assembly according to yet another embodiment of this invention.

Figure 5 is a partially exploded elevational view of the embodiment of Figure 4.

Figure 6 is an elevational view of the embodiment of Figure 4 showing the electrode and angle of insertion assemblies and a coupler.

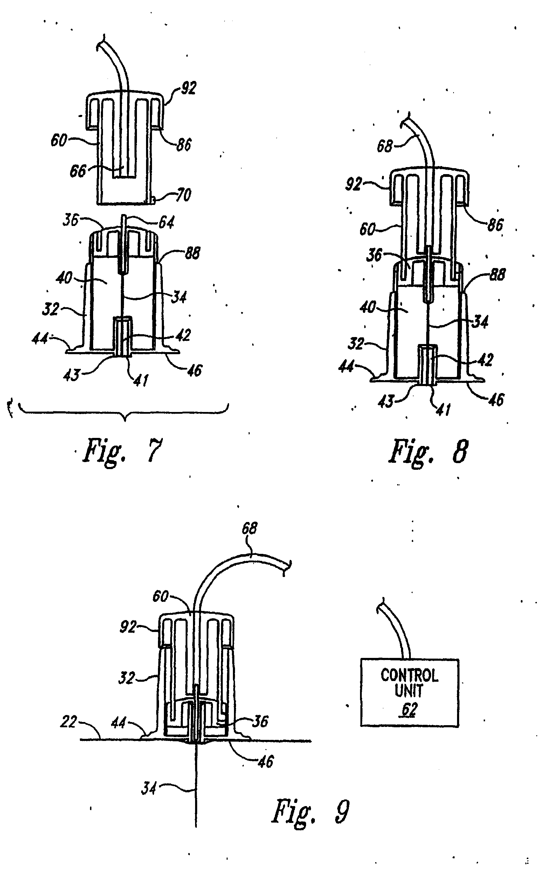

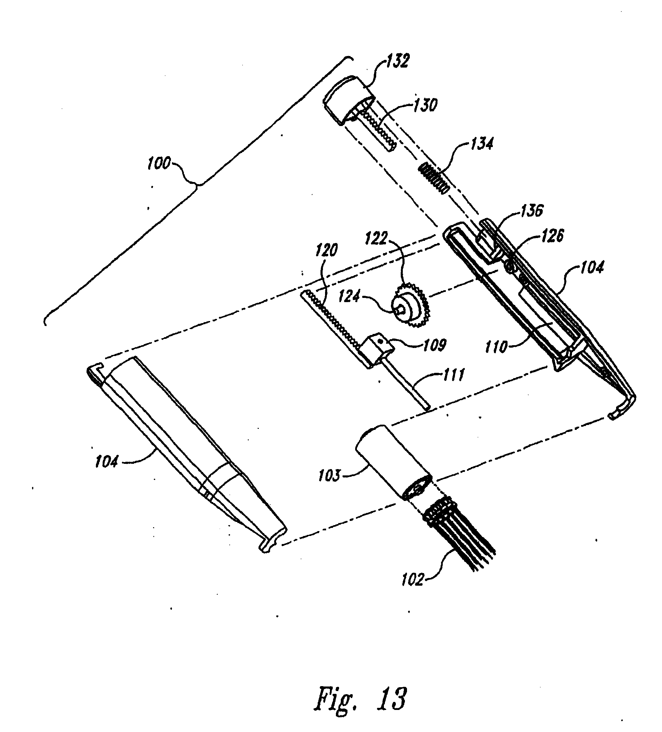

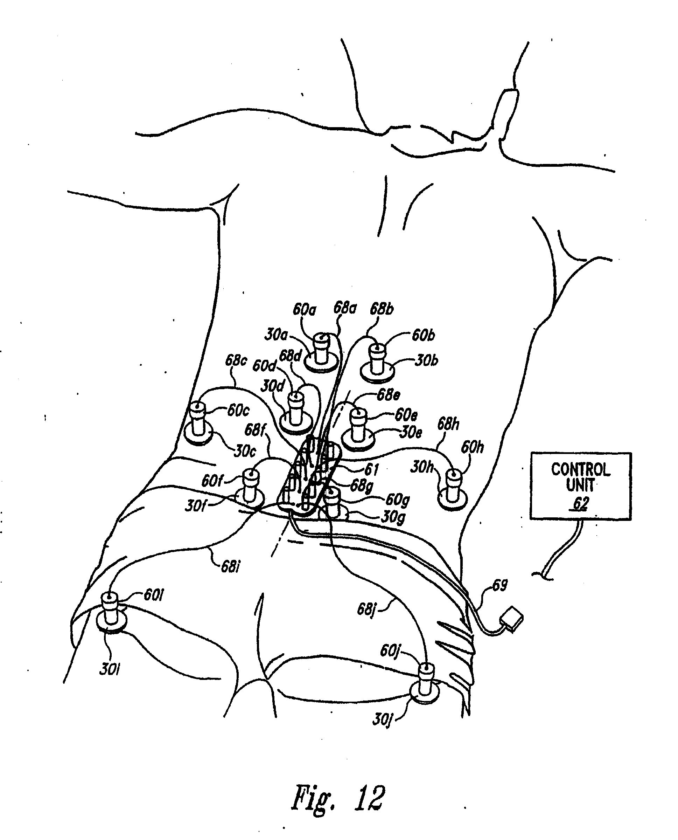

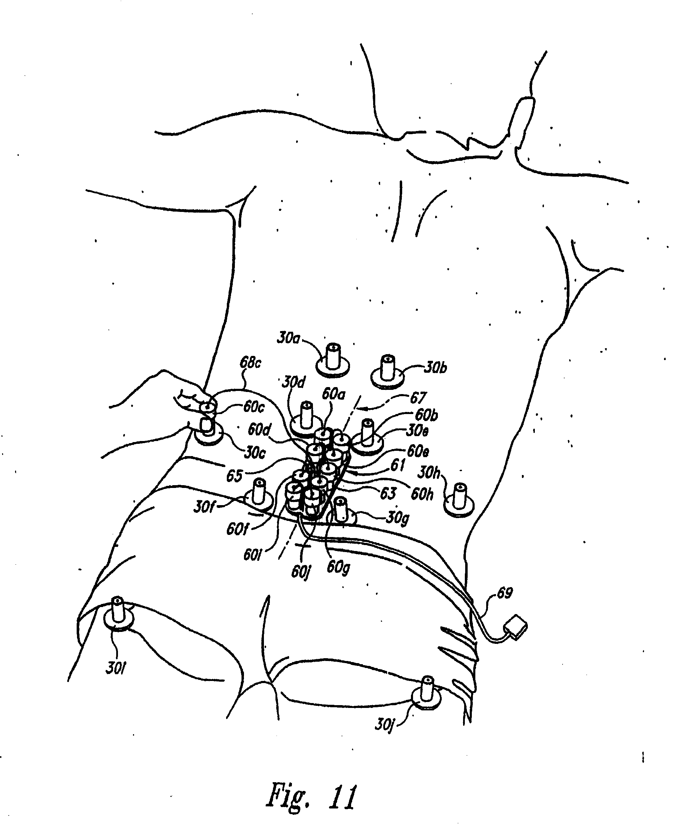

Figure 7 is a sectional view of the embodiment of Figure 4 showing the electrode and angle of insertion assemblies and a coupler.

Figure 8 is a sectional view of the embodiment of Figure 4 showing the coupler in engagement with the electrode and angle of insertion assemblies prior to insertion of the electrode into a patient's tissue.

Figure 9 is a sectional view of the embodiment of Figure 4 with the electrode in its deployed and inserted state.

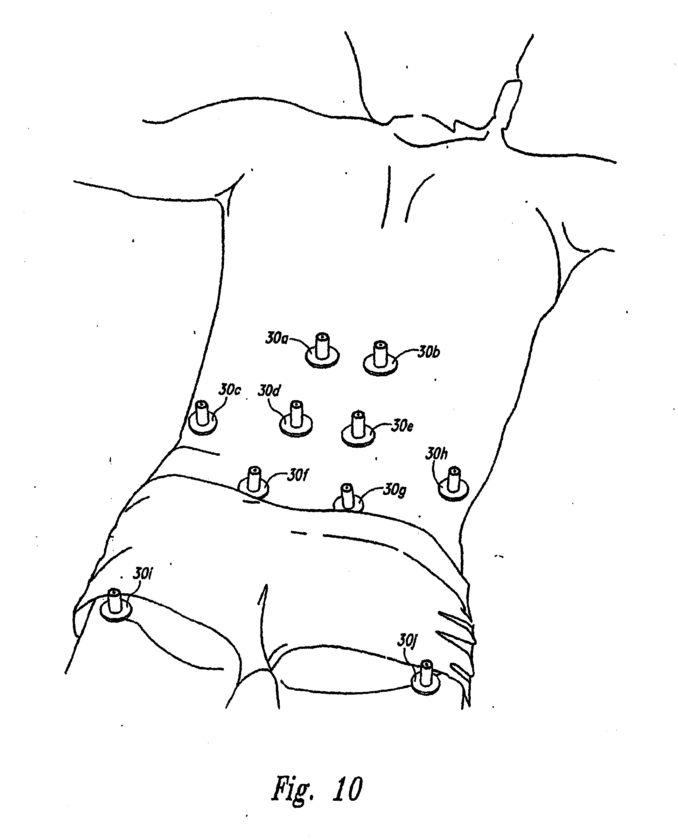

Figure 10 shows a montage for using the embodiment of Figure 4 to treat low back pain with the electrodes in a partially deployed but uninserted state.

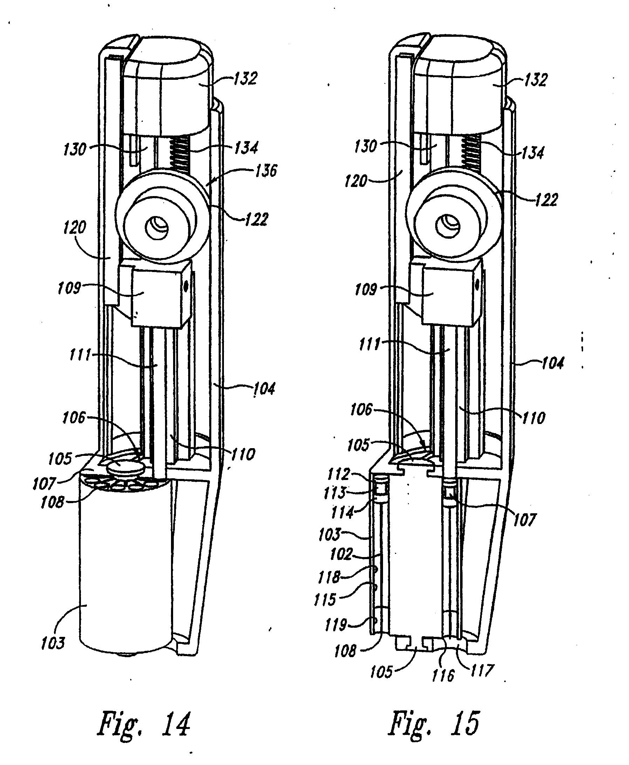

Figure 11 shows the electrode montage of Figure 10 at the beginning of the electrode insertion step.

Figure 12 shows the electrode montage of Figure 10 with the electrodes deployed, inserted and attached to a control unit to provide electrical therapy to the patient

Figure 13 is an exploded view of an electrode introducer and angle of insertion assembly of yet another embodiment of the invention.

Figure 14 is a partial sectional view of the introducer and angle of insertion assembly of Figure 13.

Figure 15 is a sectional view of the introducer and angle of insertion assembly of Figure 13.

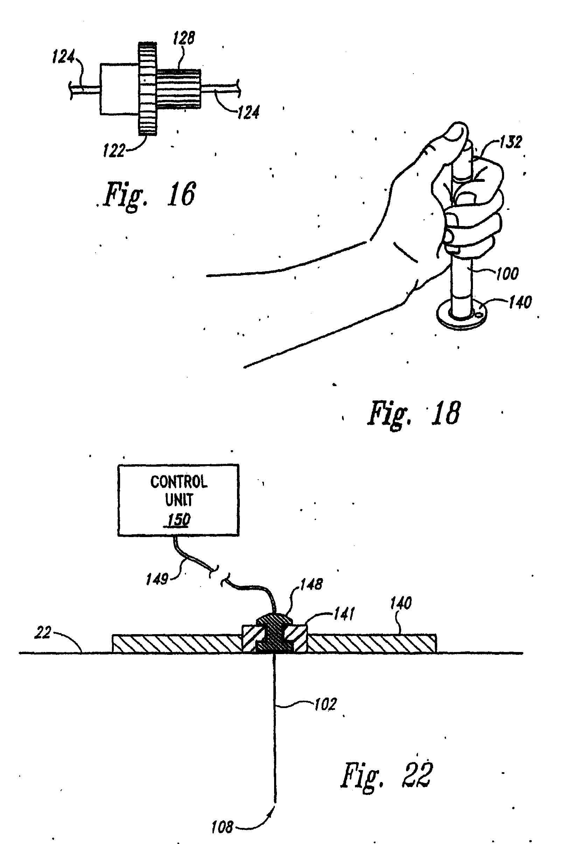

Figure 16 is an elevational view of gear assemblies of the introducer and angle of insertion assembly of Figure 13.

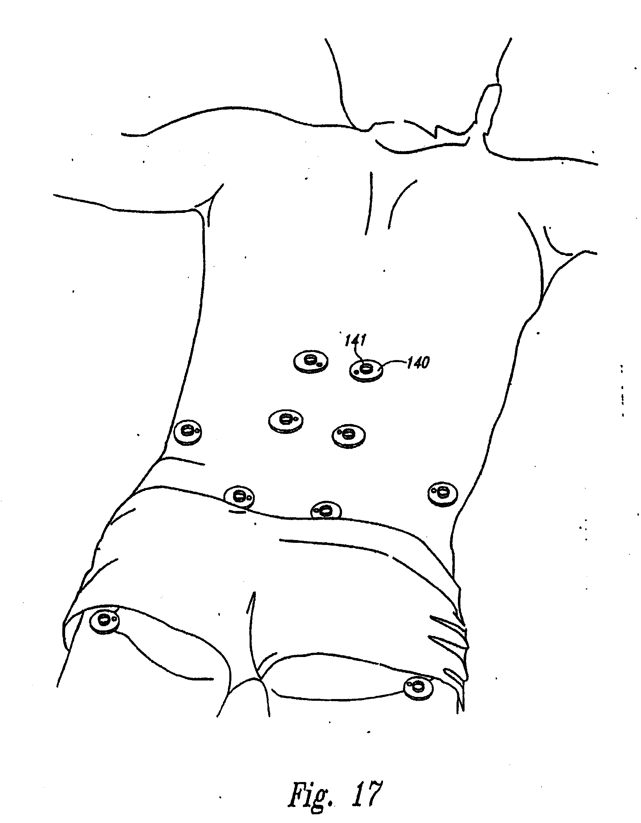

Figure 17 shows part of the electrode assembly of the embodiment of Figures 13-16 in a montage used for treating low back pain using PNT.

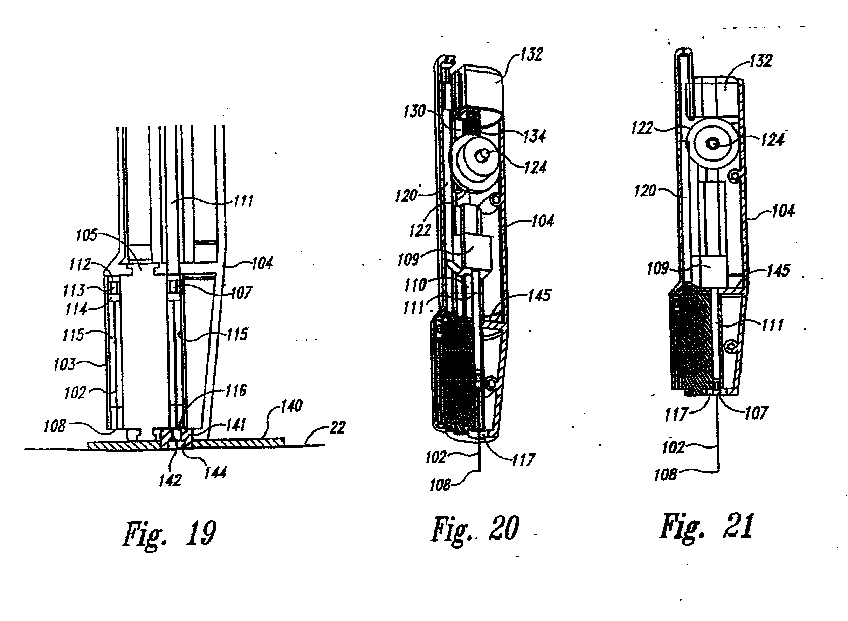

Figure 18 is an elevational view showing the introducer of Figure 13 in the process of deploying an electrode.

Figure 19 is a sectional view showing the introducer of Figure 13 in the process of deploying an electrode, prior to insertion of the electrode.

Figure 20 is a sectional view showing the introducer of Figure 13 in the process of deploying an electrode, during insertion of the electrode.

Figure 21 is a sectional view showing the introducer of Figure 13 in the process of deploying an electrode, also during insertion of the electrode.

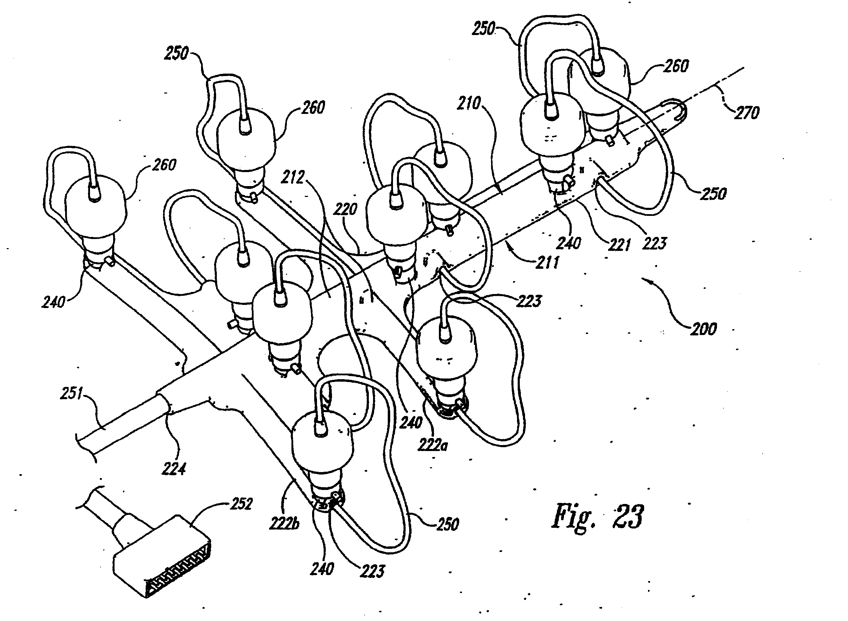

Figure 22 is a sectional view of an inserted electrode assembly of the embodiment of Figures 13-16.

Figure 23 is a partially schematic, top isometric view of a coupler support in accordance with an embodiment of the invention.

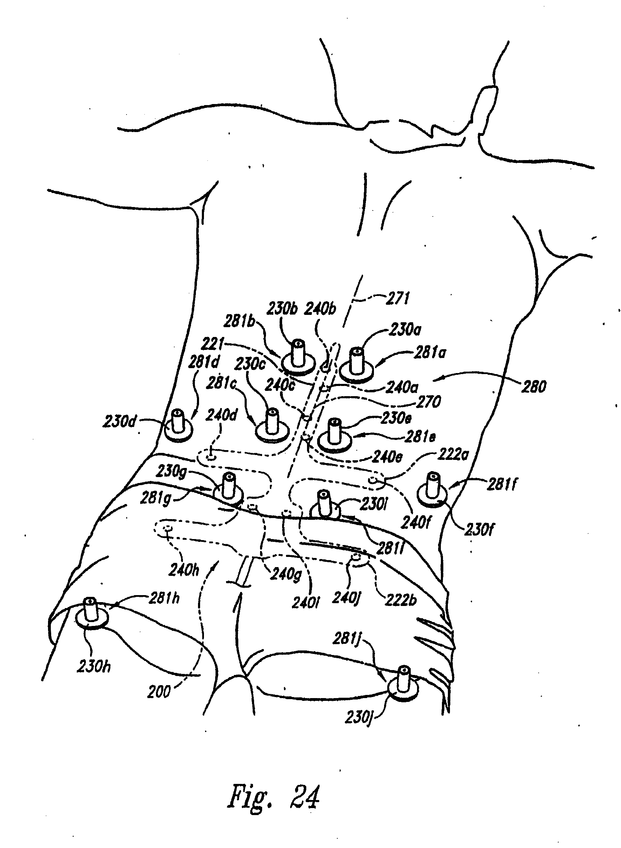

Figure 24 is a partially schematic, top isometric view of a coupler support positioned on the back of a recipient in accordance with another embodiment of the invention.

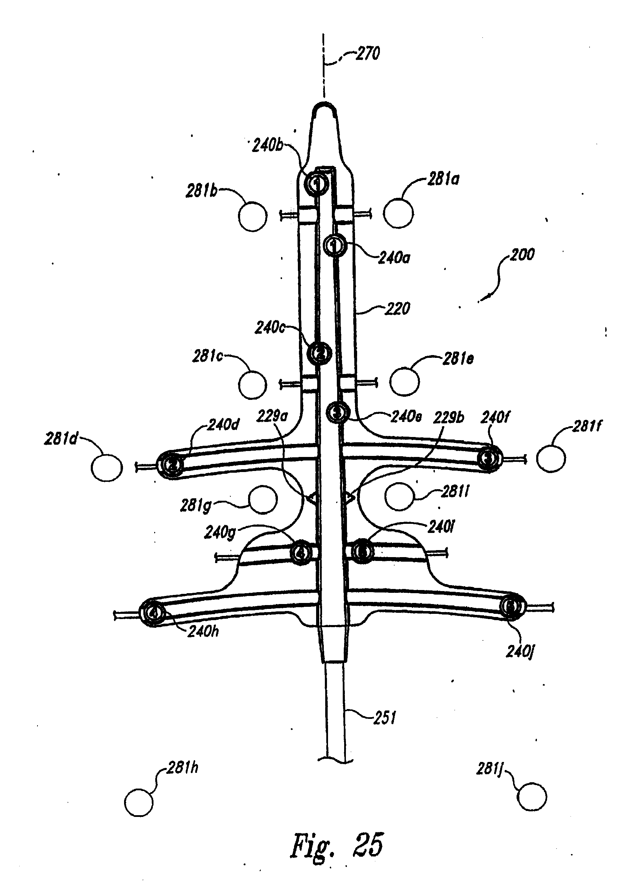

Figure 25 is a partially schematic, top plan view of a support member positioned near coupling positions in accordance with another embodiment of the invention.

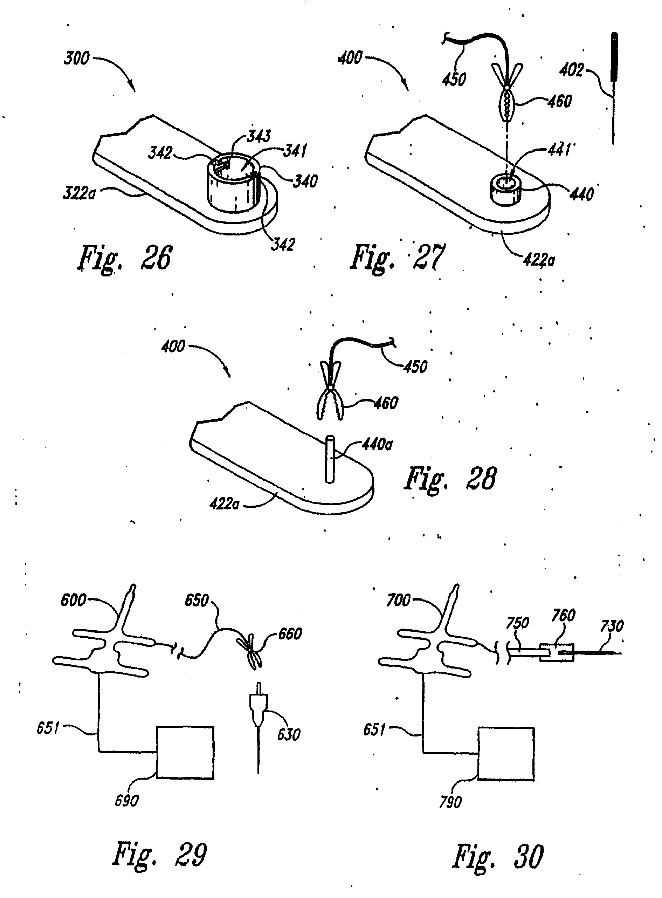

Figure 26 is a top isometric view of a portion of a coupler support configured to support a coupler in accordance with another embodiment of the invention.

Figure 27 is a top isometric view of a portion of a coupler support having an aperture configured to receive a clamp-type coupler in accordance with still another embodiment of the invention.

Figure 28 is a top isometric view of a portion of a coupler support having a post configured to be clamped by a clamp-type coupler in accordance with yet another embodiment of the invention.

Figure 29 is a partially schematic illustration of an arrangement that includes a coupler support configured to support couplers for receiving diagnostic information in accordance with yet another embodiment of the invention.

Figure 30 is a partially schematic illustration of an arrangement that includes a coupler support configured to support a plurality of couplers that administer liquid medicament in accordance with still another embodiment of the invention.

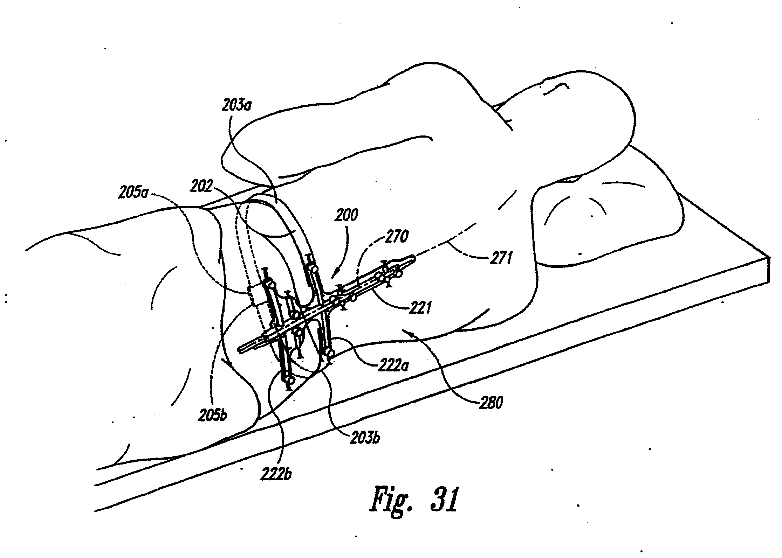

Figure 31 is a partially schematic, side isometric view of a coupler support having an attachment device configured in accordance with an embodiment of the invention for positioning the coupler support on a side-lying recipient.

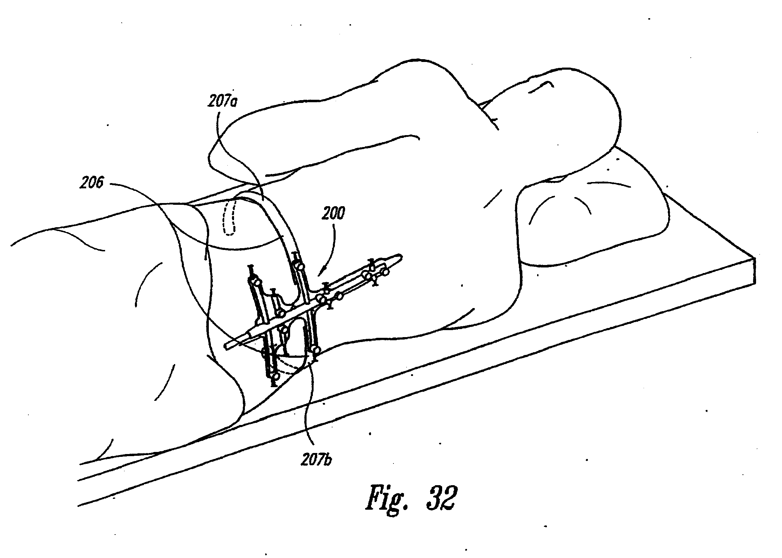

Figure 32 is a partially schematic, side isometric view of a coupler support having an attachment device configured in accordance with another embodiment for positioning the coupler support on a side-lying recipient.

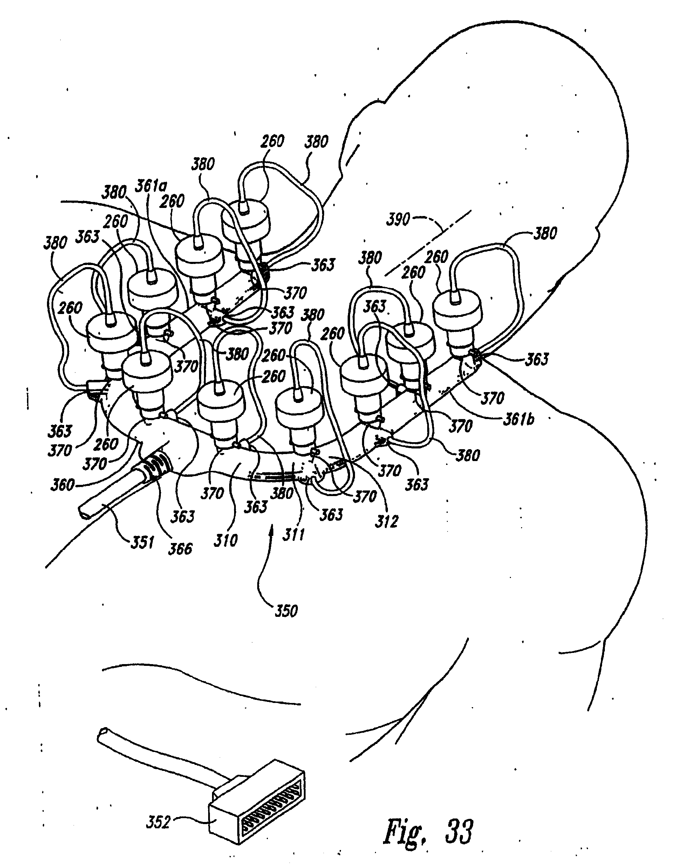

Figure 33 is a top isometric view of a coupler support configured in accordance with another embodiment of the invention positioned on a recipient.

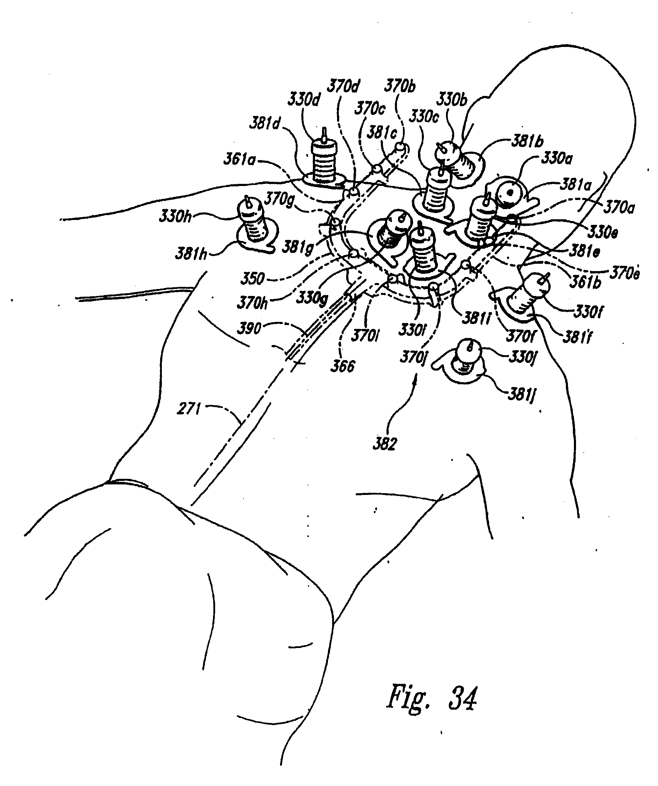

Figure 34 is a partially schematic, top isometric view of the coupler support of Figure 33 positioned near coupling positions on a back of the recipient.

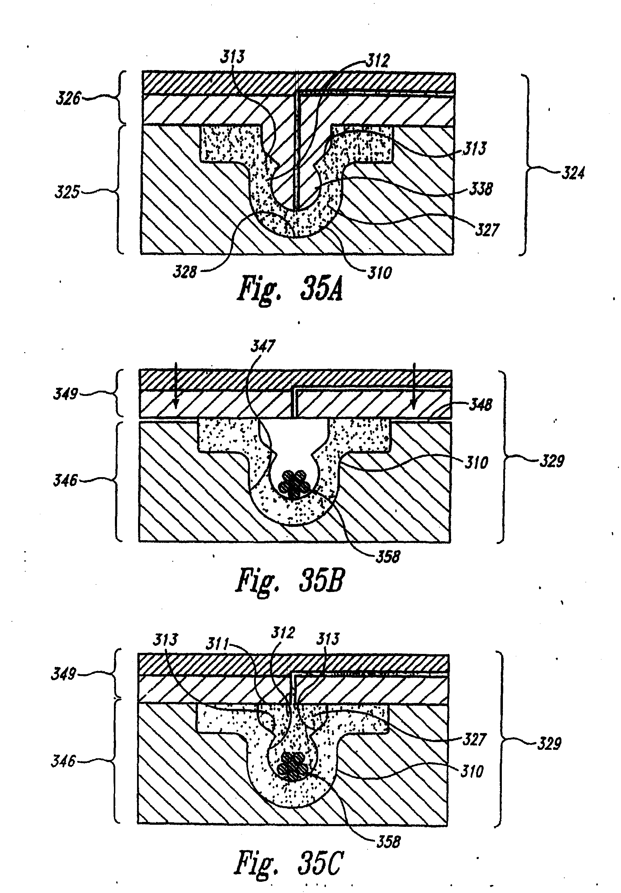

Figures 35A-C are cross-sectional end views of mold assemblies illustrating a method for manufacturing a support member or other flexible carrier in accordance with embodiments of the invention.

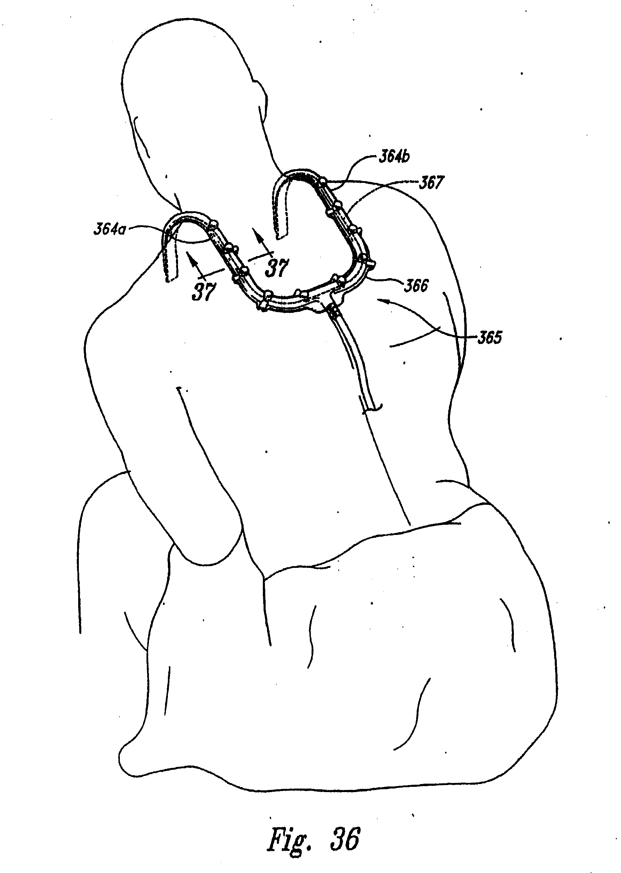

Figure 36 is a partially schematic, side isometric view of a deformable coupler support configured in accordance with a further embodiment of the invention positioned proximate to a cervical area of a recipient.

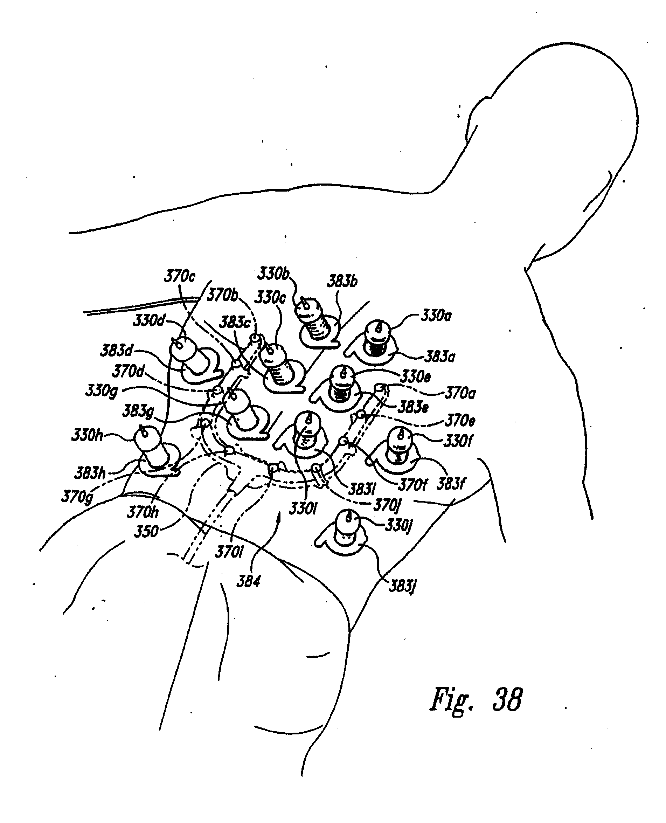

Figure 37 is a cross-sectional end view of a support member of Figure 36 taken substantially along line 37-37 in Figure 36.

Figure 38 is a partially schematic, top isometric view of the coupler support of Figure 33 positioned proximate to coupling area on a lower back region of the recipient.

### DETAILED DESCRIPTION

Percutaneous electrical therapy systems, such as PNT systems, deliver electric current to a region of a patient's tissue through electrodes that pierce the skin covering the tissue. The electric current is generated by a control unit external to the patient and typically has particular waveform characteristics such as frequency, amplitude and pulse width. Depending on the treatment or therapy being delivered, there may be one electrode containing both a cathode and an anode or a plurality of electrodes with at least one serving as a cathode and at least one serving as an anode.

The electrode has a sharp point not only to facilitate insertion through the patient's skin but also to enhance local current density during treatment. The placement and location of the electrode point is therefore an important aspect of the therapy. The electrodes must also be properly coupled to the control unit to form a complete circuit for delivering therapeutic electric current to the patient.

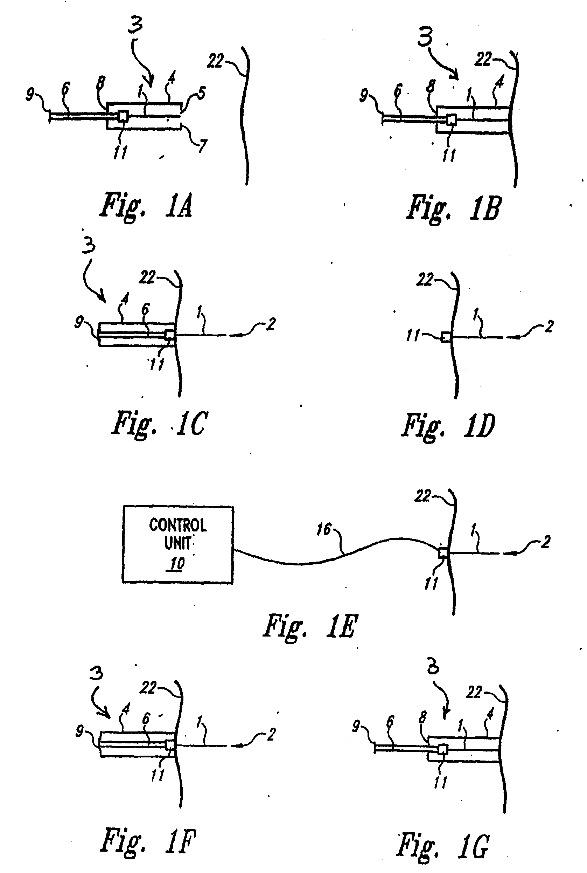

Figures 1A-G are block diagrams showing deployment and use of a percutaneous electrical therapy system and electrode assembly in accordance with an embodiment of the invention. As shown in Figures 1A and 1B, the system can include an electrode 1 having a sharp point 2 at its distal end and a housing 4 surrounding at least the sharp point 2 when the electrode 1 is in its undeployed and uninserted states. The undeployed and uninserted states include pre-deployment and post-deployment states of the electrode 1. The housing 4 can have an aperture 5 at its distal end. An actuator 6 can interact with a handle 11 at the proximal end of the electrode 1 as shown to move the electrode 1.

Deployment of the electrode assembly can include the steps taken to place the electrode assembly in proper position and condition for use in electrical therapy. Figure 1A shows the electrode assembly in an undeployed (pro-deployed) state. During deployment, the distal face 7 of the housing 4 is placed against a patient's skin 22, as shown in Figure 1B. This action supports the housing 4 with respect to the patient's skin, thereby controlling the angle between the housing and the patient's skin. The electrode 1 is then inserted through the aperture 5 into the tissue underlying the patient's skin by moving the actuator 6 distally, as shown in Figure 1C. As it moves, the actuator 6 (and therefore electrode 1) is supported by the housing 4 to control the angle at which the electrode 1 enters into the patient's tissue.

The actuator 6 may have a limit stop 9 element cooperating with a limit stop area 8 of the housing 4 to limit distal motion of the actuator 6 and to control the depth of insertion of the sharp point 2 of the electrode 1. In one embodiment, for example, when the electrical therapy system is used to provide percutaneous neuromodulation therapy, the predetermined electrode depth is 3 cm. Other electrode depths may be used, of course, depending on the intended application and therapy.

After insertion, the housing 4 and the actuator 6 (which have heretofore acted as an electrode introducer) can be removed, as shown in Figure 1D. The electrode 1 can be connected to a control unit 10 via a conductor or a cable 16. For use with PNT, the control unit 10 can supply a current-regulated and current-balanced waveform with an amplitude of up to approximately 20 mA, a frequency between approximately 4 Hz and 50 Hz, and pulse width of between approximately 50 µsec and 1 msec. Other electrical waveforms having other parameters may be used, of course, depending on the therapy to be provided. Also, while Figure 1E shows only one electrode connected to the control unit, it should be understood that a plurality of electrodes may be connected to a single control unit.

After completion of the electrical therapy, the electrode assembly can be undeployed. In an embodiment shown in Figure 1F, the aperture 5 of the housing 4 is placed over the handle portion 11 of electrode 1. The housing 4 may be the same housing used to deploy and insert the electrode (*i*.*e*., the electrode introducer), or it may be an entirely different assembly (*e.g*., an electrode remover). The sharp point 2 of the electrode 1 is then drawn into the housing 4 of sharp point protection assembly 3 by moving actuator 6 proximally, as shown in Figure 1G.

Figures 2A-E are block diagrams showing another embodiment of the invention. In one aspect of the embodiment, the control unit 10 is connected to the electrode 12 within an electrode assembly 13 via the conductor 16. As above, for use with PNT, the control unit 10 can supply a current-regulated and current-balanced waveform with an amplitude of up to approximately 20 mA, a frequency between approximately 4 Hz and 50 Hz, and pulse width of between approximately 50 µsec and 1 msec. In other embodiments, the control unit 10 can supply electrical current having other characteristics. As shown in its undeployed state in Figure 2A and in its uninserted stated in Figure 2B, the system can include a housing 18 surrounding the sharp point 20 of an electrode 12 when the electrode point 20 has not yet been inserted through the patient's skin 22.

To begin deployment, a distal face 21 of the housing 18 is placed against the patient's skin 22, as shown in Figure 2B. As with the previous embodiment, that action supports the housing 18 with respect to the patient's skin, thereby controlling the angle between the housing and the patient's skin. The sharp point 20 of the electrode 12 is then inserted through an aperture 24 into the tissue underlying the patient's skin by moving an actuator 19 distally, as shown in Figure 2C. As it moves, the actuator 19 (and therefore the electrode 12) is supported by the housing 18 to control the angle at which the electrode enters into the patient's tissue.

The actuator 19 may be part of the electrode assembly 13 or a separate component of the system. The actuator 19 may also have a limit stop element 23 that cooperates with a limit stop area 17 of the housing 18 to limit distal movement of the actuator 19, thereby controlling the depth of insertion of the electrode 12. In one embodiment, for example, when the electrical stimulation system is used to provide percutaneous neuromodulation therapy, the predetermined electrode depth is approximately 3 cm, although other electrode depths may be used depending on the application. The control unit 10 may then provide the appropriate therapy to the patient through the electrode 12 and any other electrodes connected to it.

During undeployment, the actuator 19 can draw the electrode 12 back proximally into the housing 18. After the electrode 12 is removed from the patient's skin, the housing 18 of a sharp point protection assembly 14 once again surrounds the sharp point 20 of the now uninserted electrode 12, as shown in Figures 2D and 2E. The actuator 19 helps that operation to occur without ever exposing the sharp point 20 of the electrode 12 when the sharp point 20 is no longer in the patient. In fact, the operator of the electrode assembly never sees the sharp point 20 of the electrode 12. Thus, the sharp point protection assembly 14 shields the potentially contaminated portion of the undeployed electrode 12 and protects the patient's caregiver or other bystander from unintended contact with the sharp point 20 of the electrode 12 before, during and after electrical therapy.

While Figures 2A-E show the electrode connected to the control unit prior to deployment and insertion of the electrode into the patient's skin, the connection between the control unit and the electrode could be made during deployment or after insertion. Also, while Figures 2A-E show only one electrode connected to the control unit, it should be understood that a plurality of electrodes may be connected to a single control unit, as called for by the desired electrical stimulation treatment.

To use the percutaneous electrical therapy systems of Figures 1A-G and Figures 2A-E to treat a patient, one or more electrodes are inserted through the patient's skin into the underlying tissue. As an example, to treat low back pain using PNT with unipolar electrodes, an array or montage such as that shown in Figure 3 may be used. The "T12" - "S 1" designations refer to the patient's vertebrae. The control unit or generator supplies current pulses between pairs of electrodes for durations of a few minutes to several hours, preferably delivering the current-regulated waveform described above. Thirty-minute treatments are recommended in the Ghoname *et al*. low back pain treatment articles.

Figures 4-12 show a system in accordance with another embodiment of this invention. An electrode assembly 30 can include a base 32, an electrode 34, and a plunger or actuator 36. The base 32 can have a flange or flared end 44 that is adapted to make contact with a patient's skin. The base 32 may be formed from any suitable polymer or metal, such as a high-density polyethylene (HDPE). The base 32 can be opaque so that the electrode 34 cannot be seen by a needle-shy patient.

The actuator 36 fits within a housing portion 40 of the base 32 in a slidable arrangement. A locking assembly can prevent relative movement between the actuator 36 and the housing 40 of the base 32. In one embodiment, the locking assembly of the actuator 36 has integrally-formed resilient detents 48 on its exterior cylindrical surface. In the undeployed state of the electrode assembly 30, the detents 48 mate with corresponding openings 50 in the base 32 to hold the actuator 36 and the base 32 in place with respect to each other to prevent the electrode 34 from moving outside of the protective housing 40 of the base 32, thereby providing sharp point protection. In other embodiments, mechanisms other than the detent and opening arrangement may be used to hold the actuator and base in place.

In one embodiment, the electrode 34 can include a 3-cm long 32-gauge stainless steel needle. Other sizes and materials may be used for the electrode 34, of course, without departing from the scope of the invention. The actuator 36 can be formed from HDPE as well, although other suitable materials may be used.

The electrode 34 can have a larger-diameter handle 52 at its proximal end. The handle 52 can fit within a channel 54 formed within the actuator 36. The channel 54 can have a narrow opening 56 at its distal end, with a diameter slightly larger than the diameter of the electrode 34 but narrower than the diameter of the handle 52 to hold the electrode 34 in place within the actuator 36 after initial manufacture and assembly. In the undeployed state shown in Figure 7, the sharp point 38 of the electrode 34 is disposed within the housing portion 40 of the base 32, specifically, within a narrow channel 42 of the housing 40.

To deploy one or more electrode assemblies on a patient in order to provide electrical stimulation therapy (such as PNT), the distal surface 46 of the flange portion 44 of the base 32 can be mounted on the desired site on the patient's skin, preferably with a compressible adhesive pad (not shown) surrounding a ring 43 extending downward from a surface 46 around an aperture 41 formed at the distal end of the channel 42, although other means of attaching the base 32 to the patient may be used as appropriate. This action aligns the base 32 with respect to the patient's skin. The flange portion 44 of the base 32 provides extra stability for the electrode assembly during electrode insertion and use.

A coupler or actuator tool 60 can be used to both insert the electrode and connect the electrode electrically with a control unit 62. The coupler 60 and the electrode assembly 30 can also interact to provide the sharp point protection assembly of the embodiment. When the distal end of the coupler 60 is placed against the proximal ends of the base 32 and the actuator 36, the exposed proximal end 64 of the electrode handle 52 makes electrical contact with a contact surface 66 within the coupler 60. The contact surface 66, in turn, can be electrically connected to the control unit 62 via a cable or other conductor 68.

The coupler 60 can have two oppositely disposed pegs 70 extending outwardly from the distal portion of its cylindrically surface. The pegs 70 can mate with two corresponding slots 72 in the actuator 36 and with two corresponding grooves 74 in the base 32. The second of the two slots 72 and the second of the two grooves 74 are each opposite the slot 72 and groove 74, respectively, shown in Figures 4 and 5. When connecting the coupler 60 to the electrode assembly 30, the pegs 70 move along longitudinal portions 76 of the slots 72 and along longitudinal portions 78 of the grooves 74. Concurrently, the exposed distal end 64 of the electrode handle 52 begins to make sliding contact with the contact surface 66 of actuator tool 60 to create the electrical connection between the coupler 60 and the electrode 32.

The coupler 60 can be rotated clockwise (looking down on the assembly), after the pegs 70 reach the end of the longitudinal portions 76 and 78. Accordingly, the pegs 70 move into short circumferential portions 80 and 82, respectively, of the slots 72 and the grooves 74. The length of the circumferential portions 80 of the slots 72 is less than the length of the circumferential portions 82 of the grooves 74. Continued movement of the pegs 70 along the circumferential portions 82 will therefore move the pegs 70 against the ends 81 of the circumferential slots 80. Further clockwise rotation of the coupler 60 will cause the actuator 36 to rotate clockwise as well, thereby moving the detents 48 out of the openings 50 and allowing the electrode 34 and the actuator 36 to move with respect to the base 32.

Second longitudinal portions 84 of the grooves 74 can be formed in the base 32 at the end of the circumferential portions 82. Movement of the pegs 70 distally along the second longitudinal portions 84 pushes the pegs 70 against the distal edges of the circumferential slot portions 80, thereby moving the actuator 36 and the electrode 34 in a controlled fashion distally toward the patient's skin 22.

As it moves, the electrode 34 passes through the channel 42, and the sharp point of the electrode 34 moves out through the aperture 41. The channel 42 and the actuator 36 provide axial support to the electrode 34 during this forward movement and also, along with the support provided by the flange 44, provide entry angle guidance to the electrode 34. In addition, downward pressure on the patient's skin during electrode deployment can compress the compressible adhesive pad and press the ring 43 against the patient's skin 22, which helps ease electrode entry through the skin and also lessens the insertion pain experienced by the patient.

The alignment of the base 32 with respect to the patient's skin and the controlled movement of the actuator 36 and the electrode 34 within the base 32 can control the angle at which the electrode enters the tissue underlying the patient's skin. Distal movement of the electrode 34 and its actuator within the base 32 can continue until a distal surface 86 of a cylindrical cap portion 92 of the coupler 60 meets an annular surface 88 of the housing 40. At this point, the sharp point 38 of the electrode 34 has extended a predetermined depth into the tissue underlying the patient's skin. In one embodiment, the predetermined depth is approximately 3 cm, and the depth can have other values depending on the treatment to be performed.

The electrode assembly 30 can also include a deployed electrode holding mechanism. In one aspect of the embodiment, an interference fit between the inner surface of channel 42 and the outer surface 55 of channel 52 performs the function.

Electrical stimulation treatment may begin once the electrodes have been deployed and inserted. The control unit 62 can supply stimulation current to the electrodes, *e*.*g*., in the manner described in the Ghoname *et al*. articles. The electrical waveform provided by the control unit depends on the application. For example, in one embodiment, the control unit 62 can provide a current-regulated and current-balanced waveform with an amplitude of up to approximately 20 mA, frequency between approximately 4 Hz and 50 Hz, and pulse width of between approximately 50 µsec and 1 msec. In other embodiments, the control unit 62 can provide electrical current at other frequencies.

The interaction of the coupler 60 and the base 32 can provide stability to the electrode 34 and its electrical connection to the control unit during treatment by holding the electrode in place, by providing strain relief for tugging forces on the cable 68, and by providing a robust mechanical connection. It should also be noted that in one aspect of those embodiments, the sharp point of the electrode 34 is not exposed to the operator or to any other bystander at any point during deployment and use of the electrode assembly.

After treatment has been completed, the electrode may be removed from the patient. To do so, the coupler 60 can be moved proximally away from the patient. As the pegs 70 move proximally along the longitudinal portions 84 of the grooves 74, the pegs 70 push against the proximal edges of the actuator's circumferential slot portions 80, thereby moving the actuator 36 and the electrode 34 proximally as well. When the pegs 70 reach the proximal end of the longitudinal groove portions 84, the sharp end 38 of the electrode 34 is out of the patient and safely inside the housing 40 of the base 32. Counterclockwise movement of the coupler 60 moves the pegs 70 along the circumferential portions 80 and 82 of the slot 72 and the groove 74, respectively. Because the circumferential portion 80 is shorter than the circumferential portion 82, the counterclockwise movement will turn the actuator 36 counterclockwise.

At the limit of the counterclockwise movement, the detents 48 move back into the openings 50 to prevent further movement of the electrode and the actuator with respect to the base 32. Further distal movement of the coupler 60 moves the pegs 70 distally along the longitudinal portions 76 and 78 of the slot 72 and the groove 74, respectively, to disconnect the coupler 60 from the electrode assembly 30. The base 32 can then be removed from the patient.

Figures 10-12 show the use of the electrode and sharp point protection assemblies of Figures 4-9 to treat low back pain using PNT. As shown in Figure 10, ten electrode assemblies 30a-j are arranged in a montage on the patient's back and attached with adhesive. Next, ten couplers 60a-j are attached to the ten electrode assemblies 30a-j. In one embodiment (shown in Figure 11), the couplers 60a-j are supported or carried prior to deployment by a coupler support 61 (Figure 12). In one aspect of the embodiment, the coupler support 61 can include a generally flat, rigid support member 63 having ten engagement members 65 positioned at corresponding coupler locations of the support member 63. Each engagement member 65 can be configured to removably support or carry one of the couplers 60. For example, each engagement member 65 can include a post projecting upwardly from the support member 63 to be removably received in a corresponding axial aperture of the coupler 60. As shown in Figure 12, each coupler 60 can be connected to the support member 63 with an individual cable 68a-j. The individual cables 68a-j can be bundled together to form a link 69 (such as a multi-wire cable) that provides electrical communication between the couplers 60 and a control unit 62.

In another aspect of the embodiment, an arrangement of the engagement members 65 on the support member 63 can correspond to an arrangement of the electrode assemblies 30a-j on the patient's back. For example, when the electrode assemblies 30a-j are connected to the patient at ten sites arranged in two rows on each side of the patient's spine, the engagement members 65 can be arranged in two rows, one on each side of a central axis 67 (Figure 11) that can be aligned with the patient's spine. Accordingly, the arrangement of the engagement members 65 can guide the practitioner to connect each coupler 60 to the proper electrode assembly 30. Because each electrode assembly 30 is paired with another to define a complete electrical circuit (with one electrode serving as an anode and an adjacent electrode serving as a cathode), it can be important to correctly match the individual cable 68 with the corresponding electrode assembly. For example, if a given electrode assembly 30 serving as an anode is inadvertently placed too distant from the corresponding electrode assembly 30 serving as a cathode, the current applied to the electrode assemblies may be too weak to be effective. Furthermore, when the characteristics of the electrical signals supplied to each circuit are controlled separately, it may not be clear which circuit is being controlled if the couplers 60 are attached to the wrong electrode assemblies. Accordingly, the coupler support 61 can increase the effectiveness of the electrical stimulation therapy by reducing the likelihood that the couplers 60 will be incorrectly deployed. In other embodiments, the coupler support 61 can have other configurations and can support couplers having other configurations, as described below with reference to Figures 23-30.

Once each electrode assembly 30 has been actuated by its respective coupler 60 to insert an electrode into the patient's tissue (as shown in Figure 12), the control unit 62 provides electrical signals to treat the patient. As described above, half the electrodes (*e*.*g*., assemblies 30b, 30d, 30g, 30h and 30i) can serve as anodes, and the other half as cathodes. In one embodiment, the control unit 62 can provide a current-regulated and current-balanced waveform with an amplitude of up to approximately 20 mA, frequency between approximately 4 Hz and 50 Hz, and pulse width of between approximately 50 µsec and 1 msec. to treat the patient's low back pain using PNT.

Figures 13-22 illustrate an apparatus in accordance with another embodiment of the invention. In one aspect of this embodiment, an electrode introducer and an alignment member mounted on the patient's skin provide an electrode angle of insertion assembly controlling the electrode's angle of entry into the patient's tissue. In a further aspect of this embodiment, an electrode introducer 100 shown in Figures 13-16 and 19-21 can insert multiple electrodes. It should be understood that the principles of this invention could be applied to an introducer designed to hold and insert any number of electrodes.

Twelve electrodes 102 are disposed within a magazine 103 rotatably mounted within a housing 104. In one embodiment, the housing 104 is a two-part injection molded polystyrene assembly. As shown in Figure 14, the magazine 103 rotates about a hub 105 mounted on supports formed in the housing 104. A leaf spring 106 mates with one of twelve radial grooves 108 formed in the magazine 103 to form a twelve-position ratchet mechanism for the rotatable magazine 103 in the housing 104.

The magazine 103 can have twelve electrode chambers 115 arranged radially about the hub 105. When the introducer 100 is completely full, each chamber 115 contains one electrode 102. The diameter of an upper portion 118 of the chamber 115 is sized to form an interference fit with the wider portions 112 and 114 of an electrode handle portion 107 of the electrode 102. A lower wide portion 114 of the electrode 102 can be formed from a compressible material. The diameter of a lower portion 119 of the chamber 115 is slightly larger so that there is no interference fit between the lower portion 119 and the electrode handle 107, for reasons explained below. Each time the leaf spring 106 is within a groove 108, the opening 106 of a magazine chamber 115 is lined up with the aperture 117 of the introducer 100, as shown in Figures 14 and 15.

A slide member 109 is disposed on a rail 110 formed in the housing 104. Extending longitudinally downwardly from the slide member 109 is a drive rod 111, and extending longitudinally upwardly from the slide member 109 is a gear rack 120. The teeth of the gear rack 120 cooperate with the teeth on a rotational gear 122 mounted about a shaft 124 extending into a shaft mount 126 formed in the housing 104. A second set of teeth are mounted on a smaller diameter rotational gear 128 (shown more clearly in Figure 16) which is also mounted about the shaft 124. The gears 122 and 128 rotate together about the shaft 124.

The teeth of the smaller diameter gear 128 mesh with the teeth of a second gear rack 130 extending from a longitudinally-movable actuator 132. A spring 134 mounted between the actuator 132 and a spring platform 136 biases the actuator 132 away from the housing 104.

To deploy the electrode assembly of this embodiment, a flexible and compressible annular patch 140 is placed on the patient's skin at the desired site, preferably with an adhesive (not shown). For example, to treat low back pain using PNT, the arrangement or montage shown in Figure 17 may be used. In this montage, five electrodes serve as cathodes and five serve as anodes.

As shown in Figures 19 and 20, the patch 140 has an annular rigid member 141 disposed in its center and extending upwardly from it. The rigid member 141 has a smaller diameter opening 142 leading to a larger diameter opening 144. The diameter of the opening 142 is slightly smaller than the lower wide portion 114 of the handle portion 107 of the electrode 102 and slightly larger than the diameter of the central portion 113 of the handle portion 107 of the electrode 102.

After the patch 140 is in place, the distal end of the introducer 100 is placed against the patch 140 so that the introducer aperture 117 surrounds the upwardly extending portion of rigid patch member 141, as shown in Figure 18. This interaction aligns the opening 116 of one of the introducer's magazine chambers 115 with the opening 142 of the rigid member 141 and helps control the electrode's angle of entry, as shown in Figure 19. Downward pressure on the introducer 100 compresses the patch 140, thereby causing the upper surface of the rigid member 141 to engage a lower surface of the magazine 103, and pressing the rigid member 141 downward into the patient's skin 22. The pressure on the patient's skin around the insertion site can reduce the pain caused by inserting the electrode.

Depressing the actuator 132 moves the gear rack 130 distally, which causes the gears 128 and 122 to rotate. Because the diameter and tooth count of the gear 128 differ from the diameter and tooth count the gear 122, the gear rack 120 moves longitudinally a much greater distance than the corresponding longitudinal movement of the gear rack 130. That feature enables the electrode to be inserted its required distance into the patient's skin using only a comparatively small movement of the operator's thumb. Distal movement of the gear rack 120 is guided by the movement of the slide member 109 along the rail 110.

As the slide member 109 moves distally, the drive rod 111 moves into a magazine chamber 115 until the distal end of the drive rod 111 engages the top surface of the electrode's handle portion 107. As shown in Figure 20, further distal movement of the drive rod 111 pushes the electrode 102 downwardly so that the sharp point 108 of the electrode 102 leaves the introducer housing and enters the patient's skin 22 and the tissue beneath the skin. The chamber 115 provides axial stability to the electrode 102 during insertion.

When the top portion 112 of the electrode handle portion 107 leaves the smaller diameter portion 118 of the magazine chamber 115, it enters the larger diameter portion 119 of the chamber 115. At this point (shown in Figure 21), because the diameter of chamber portion 119 is wider than the diameter of the electrode handle 107, the electrode is no longer attached to the introducer 100.

Continued downward movement of the actuator 132 and the drive rod 111 pushes the lower larger diameter portion 114 of the electrode handle 107 through the smaller diameter portion 142 of rigid member 141 by compressing the handle portion 114. Further downward movement pushes the handle portion 114 into the larger diameter portion 144 of the rigid member 141 so that the rigid member's smaller diameter portion lies between the larger diameter portions 112 and 114 of the electrode handle 107. That interaction holds the electrode in place in the patient's tissue and helps provide depth control for electrode insertion. In the embodiment, the preferred depth of the electrode's sharp point 108 is approximately 3 cm, although the electrode may be inserted to other depths depending on the treatment to be performed. The slider member 109 also acts as a limit stop at that point when the slide member 109 engages the limit stop area 145 of the housing 104, thereby also controlling electrode insertion depth.

The magazine 103 can be rotated to a now insertion position and placed against an empty patch 140 after insertion of each electrode until all electrodes have been deployed and inserted. A suitable electrical connector 148, such as an alligator clip, can be electrically connected to the electrode 102 through an aperture (not shown) formed in the upper larger diameter portion 112 of the electrode handle 107 to provide electrical communication between a control unit 150 and the electrode 102 via a cable or other conductor 149, as shown in Figure 22. The patch 140 can provide strain relief for the electrode 102 by preventing tugging forces on the cable 149 from dislodging the electrode from the patient, thereby helping keep the electrode in place. In one aspect of the embodiment, the sharp point of the electrode is not exposed to the operator or bystander at any point during the electrode deployment, insertion and electrical therapy treatment processes.

The control unit 150 supplies stimulation current to the electrodes, e.g., in the manner described in the Ghoname *et al*. articles. Once again, the electrical waveform provided by the control unit depends on the application. For example, in an embodiment of a system providing percutaneous neuromodulation therapy, the control unit 150 can provide a current-regulated and current-balanced waveform with an amplitude of up to approximately 20 mA, frequency between approximately 4 Hz and 50 Hz, and pulse width of between approximately 50 µsec and 1 msec.

In an alternative embodiment, the lower wide portion of the electrode handle can be formed from a rigid material and can have rounded camming edges. The central annulus of patch 140 in that alternative embodiment is either compressible or has a resilient camming opening under the camming action of the electrode handle.

Figure 23 is a top isometric view of a coupler support 200 that supports or carries couplers 260 in accordance with another embodiment of the invention. In one aspect of that embodiment, the coupler support 200 includes a support member 220 and ten engagement members 240 positioned at coupler locations of the support member 220. Each engagement member 240 can removably support one of the couplers 260. In one aspect of that embodiment, the couplers 260 can be generally similar to the coupler 60 described above with reference to Figures 4-12. Alternatively, the coupler 260 can have other configurations, such as the configuration disclosed in co-pending U.S. Application No. 09/666,931, entitled "Method and Apparatus for Repositioning a Percutaneous Probe," incorporated above by reference. In other embodiments, the coupler can have other configurations, for example, those described below with reference to Figures 27-30.

When the couplers 260 are generally similar to the couplers 60 described above with reference to Figures 4-12, each engagement member 240 can have columnar or post shape and can be removably received in a downward facing aperture of the coupler 260. The engagement member 240 can extend a sufficient distance upwardly into the aperture of the coupler 260 to firmly support the coupler 260 relative to the support member 220. In other embodiments, the engagement member 240 can have other configurations (for example, when the coupler has other configurations), as described below with reference to Figures 26-30.

The coupler support 200 can include links 250 between the support member 220 and each coupler 260. In one aspect of the embodiment, the links 250 can include electrical cables to transmit electrical signals to the couplers 260 and to the patient or recipient to whom the couplers 260 are attached. In other embodiments, the links 250 can have other configurations, as described below with reference to Figures 27-30. In any of those embodiments, different links 250 can have different lengths to allow the corresponding coupler 260 to be coupled to the appropriate site on the recipient. Alternatively, each link 250 can have the same length, so long as the length is sufficient for each coupler 260 to be coupled to the proper site on the recipient. For example, in one embodiment, a single support member 220 with a single set of links 250 can be compatible with recipients ranging in height from about 4.5 feet to about 6.5 feet.

When the links 250 include electrical cables, each link 250 can enter the support member 220 at an entry attachment point 223. The links 250 can then pass through a cable channel 212 of the support member 220 and exit the support member 220 at an exit attachment point 224. The links 250 can be bundled together to form a bundled link 251 that can be attached to an electrical connector 252 for coupling to a source of electrical potential.

In one embodiment, the support member 220 can include an upper portion 210 bonded to a lower portion 211. The upper portion 210 can include the cable channel 212 and the engagement members 240. The support member 220 can be formed by molding the upper portion 210, inverting the upper portion 210, and laying a cable harness (which includes the bundled link 251 and the individual links 250) into the cable channel 212. The lower portion 211 can be attached to the upper portion 210 (for example, in an overmold process) to fix the harness into the support member 220. In other embodiments, the coupler support 200 can be formed with other techniques. In any of those embodiments, the support member 220 can include a flexible, soft durometer, bio-compatible, thermoplastic elastomeric material, such as Santoprone®, available from Advanced Elastomeric Systems of Akron, Ohio. Accordingly, the support member 220 can conform to the shape of the recipient's body, as described below with reference to Figure 24.

In a further aspect of the embodiment, the shape of the support member 220 and the positions of the engagement members on the support member 220 can be configured to aid the practitioner in connecting each coupler 260 to the correct corresponding coupling site on the recipient's body. For example, when the coupler support 200 is configured to administer electrical therapy to the recipient's back, the support member 220 can have an axial elongated portion 221 aligned with a central axis 270. The support member 220 can further include two transverse elongated portions 222 (shown as a first transverse elongated portion 222a and second transverse elongated portion 222b) arranged transverse to the central axis 270. In one aspect of the embodiment, the coupler support 200 generally and the elongated portions 221, 222 in particular can be configured to be spaced apart from corresponding coupling sites on the recipient's back, so as not to interfere with the operation of attaching the couplers 260 to the recipient. For example, in one embodiment, the axial elongated portion 221 can have a length of about 11 inches ± 0.25 inch (measured from the exit attachment point 224). The transverse elongated portions 222a, 222b can have lengths of about 6.8 inches and about 7.5 inches, respectively, ± 0.25 inch. In other embodiments, the elongated portions 221, 222 can.have other dimensions. In any of those embodiments, each coupler 260 can be positioned proximate to a corresponding coupling site to aid the practitioner in connecting the couplers with the appropriate coupling site, as described below with reference to Figure 24.

Figure 24 is a partially schematic, top isometric view of the coupler support 200 (shown in phantom lines) placed in position on the back of a recipient. For purposes of clarity, the coupler support 200 is shown schematically in Figure 24 without the couplers 260. In one aspect of the embodiment, the central axis 270 of the coupler support 200 is aligned with a body longitudinal axis 271 (such as the spine) to position the coupler support 200 proximate to a coupling area 280 on the recipient. The coupling area can be on the recipient's back (as shown in Figure 24) or, alternatively the coupling area can be on the recipient's neck, head, leg or other body part. When the coupler support 200 is in position on the coupling area 280, the elongated portions 221, 222a and 222b can flex to conform to the shape of the recipient's body in the coupling area 280. Accordingly, the coupler support 200 can be less likely to be dislodged from the recipient's body and can more accurately align the couplers 260 with the appropriate portions of the coupling area 280.

The coupling area 280 includes a plurality of coupling positions or sites 281 (shown as 281a-j) at which a corresponding plurality of electrode assemblies 230 (shown as 230a-j) are attached. In one embodiment, the electrode assemblies 230 are arranged in cathode/anode pairs with five circuits formed by electrode assembly pairs 230a and 230b; 230c and 230d; 230e and 230f; 230g and 230h; and 230i and 230j. Once the coupler support 200 is in position on the recipient's back, each engagement member 240 (shown as 240a-j) is positioned proximate to its corresponding electrode assembly 230a-j. For example, those engagement members 240 that are to be coupled with electrode assemblies 230 close to the body longitudinal axis 271 are positioned close to the central axis 270 of the coupler support 200. Those engagement members 240 that are to be coupled with electrode assemblies 230 further away from body longitudinal axis 271 are positioned further away from the central axis 270 of the coupler support 200. Accordingly, many of the couplers 260 are positioned closer to the one corresponding electrode assembly 230 to which that coupler 260 is to be connected than to any other electrode assembly. As a result, practitioners will be less likely to link the couplers 260 to the incorrect electrode assembly 230.

Figure 25 is a top plan view of the coupler support 200 with the couplers 260 removed so that the tops of the engagement members 240a-j are visible. In one aspect of the embodiment, the engagement members 240a-j can be marked to indicate which circuit the corresponding couplers 260 are connected to. For example, the engagement members 240a and 240b can be marked with a numeral "1" to indicate that the couplers 260 removed from those engagement members are connected to the recipient to form circuit number "1." An advantage of that arrangement is that if the control unit 62 (Figure 9) indicates that circuit number "1" is faulty or defective, the practitioner can easily narrow the field of potentially faulty couplers 260 to the two couplers 260 removed from engagement members 240a and 240b.

In another aspect of the embodiment, the coupler support 200 can include other features to further aid the practitioner in attaching the couplers 260 to the correct coupling site 281. For example, the engagement member 240a can be can be offset to the right side of the central axis 270 and the engagement member 240b can be offset to the left side of the central axis 270 so that the practitioner will be more likely to connect the corresponding couplers 260a, 260b (Figure 24) to the appropriate coupling site 281a, 281b. In a further aspect of the embodiment, the engagement members 240a, 240e, 240f, 240i and 240j positioned on the right side of the central axis 270 can have a different color than the engagement members 240b, 240c, 240d, 240g and 240h positioned on the left side of the central axis 270. As is also shown in Figures 24 and 25, the overall shape of the coupler support 200, and in particular, an outline defined by the positions of the engagement members 240, is generally similar to an outline defined by the positions of the coupling sites 281. In other embodiments, for example, when the coupler support 200 is configured to rest on the recipient's leg, neck or head for therapy to those regions, the outline defined by the engagement members can also correspond to the outline defined by the coupling sites. In any of those embodiments, the relative longitudinal and lateral locations of the engagement members 240 can correspond at least roughly with the relative longitudinal and lateral locations of the coupling sites 281 on the recipient's body.

To further aid the practitioner, the coupler support 200 can include alignment features 229 (shown as a first alignment feature 229a and a second alignment feature 229b). The practitioner can use the alignment features 229 for proper positioning of the coupler support 200 by aligning the alignment features 229 with corresponding coupling sites 281g and 281 i when positioning the coupler support 200 on the recipient's body. In other embodiments, the coupler support 200 can include additional alignment features that relate to other coupling sites, or the alignment features 229 can be omitted.

Figure 26 is a top isometric view of a portion of a coupler support 300 having a transverse elongated portion 322a with an engagement member 340 in accordance with another embodiment of the invention. The overall shape of the coupler support 300 can be generally similar to that described above with reference to Figures 23-25. In one aspect of the embodiment, the engagement member 340 can include an aperture 341 positioned to receive the housing of a coupler, such as the coupler 260 described above with reference to Figure 23 or the coupler 60 described above with reference to Figures 4-8. In still a further aspect of the embodiment, the engagement member 340 can include a pair of entrance slots 342 positioned to receive the pegs 70 (Figure 5) of the coupler 60, Once the pegs 70 have been moved into the entrance slots 342, the coupler 60 can be rotated clockwise to move the pegs 70 into transverse locking slots 343. Accordingly, an advantage of that arrangement is that it can securely, yet removably, engage the coupler 60.

Figure 27 is a top isometric view of a portion of a coupler support 400 having a transverse elongated portion 422a with an engagement member 440 in accordance with another embodiment of the invention. In one aspect of the embodiment, the engagement member 440 can include an aperture 441 configured to receive a clamping coupler 460, such as an alligator clip. The clamping coupler 460 can be attached directly to a percutaneous acupuncture needle 402 or another percutaneous or transcutaneous device. When the clamping coupler 460 is not attached to the needle 402, it can be removably positioned in the aperture 441 while remaining connected to an electrical link 450, such as a cable. In an alternate arrangement shown in Figure 28, the coupler support 400 can include an engagement member 440a having a post shape. In one aspect of the embodiment, the clamping coupler 460 can be clamped to the engagement member 440a to support the coupler 460 relative to the coupler support 400.

In one aspect of the embodiments described above with reference to Figures 27 and 28, the clamping coupler 460 can be attached to a percutaneous electrode, such as the electrode 102 described above with reference to Figure 22. Alternatively, the coupler 460 (or other couplers) can be attached to a transcutaneous electrical nerve stimulation system. In still a further embodiment (shown in Figure 29), a clamping coupler 660 can be clamped to a diagnostic electrode, such as a patch electrode or an EMG needle electrode 630 of the type available from SLE of South Croydon, England. A plurality of the couplers 660 (one of which is shown in Figure 29) can be connected to a support 600 (shown schematically in Figure 29) in a configuration generally similar to that described above with reference to Figure 23 and/or Figure 11. The support 600 can be connected with a bundled link 651 to a care unit 690. The care unit 690 can include a diagnostic instrument that receives electrical signals from the coupler 660, rather than providing electrical signals to the coupler 660. Accordingly, the support 600 can aid the practitioner in coupling the plurality of couplers 660 to the correct corresponding electrode 630.

Figure 30 is a partially schematic view of a coupler support 700 that can aid the practitioner in delivering medicament to a plurality of coupling sites on the recipient in accordance with another embodiment of the invention. Accordingly, the coupler support 700 can include a plurality of medicament links 750 (one of which is shown in Figure 30), such as a length of drug delivery tubing. Each link 750 can include a coupler 760 for attaching to a needle or other drug delivery device 730 inserted into the recipient. The individual medicament links 750 can be bundled together to form a bundled link 751 which is connected to a care unit 790. The care unit 790 can include a pump, drip bag, or other arrangement for providing liquid medicament to the coupler support 700 and the recipient.

A feature of embodiments of the coupler support arrangements described above with reference to Figures 11 and 23-30 is that the supports are configured to position the couplers proximate to the appropriate coupling location. Accordingly, practitioners, including relatively inexperienced practitioners, can be less likely to connect the couplers to an incorrect coupling location. An advantage of the arrangement is that the couplers can provide more reliable and/or more efficacious therapy and/or diagnostic information. A further advantage is that the practitioner can more quickly connect the couplers to the recipient, increasing the efficiency with which the practitioner can provide therapy and/or diagnostic information.

Still a further advantage of embodiments of the coupler support described above is that a single support can accommodate a wide variety of applications. For example, a single support can be used with recipients ranging in height from about 4.5 feet to about 6.5 feet, as discussed above. A single coupler support can also be used with recipients having a wide variety of body shapes. Still further, a single coupler support can be positioned on recipients having a variety of postures. For example, a single coupler support can rest on the recipient's back whether the recipient is prone, leaning over, or partially upright, while still allowing the couplers to be connected to the appropriate coupling locations.

Figure 3 1 is a partially schematic, side isometric view of the coupler support 200 held in position on a side-lying recipient with an attachment device 202 configured in accordance with an embodiment of the invention. For purposes of clarity, the coupler support 200 is shown schematically in Figure 31 without the couplers 260. In one aspect of the embodiment, the central axis 270 of the coupler support 200 is aligned with the body longitudinal axis 271 (such as the spine) to position the coupler support 200 proximate to the coupling area 280 on the recipient. The coupling area 280 can be located on the recipient's back (as shown in Figure 24) or, alternatively, the coupling area 280 can be on the recipient's neck, head, leg or other body part.

In another aspect of the embodiment, the attachment device 202 includes a first attachment portion 203a extending from the first transverse elongated portion 222a in a first direction, and a second attachment portion 203b extending from the first transverse elongated portion 222a in a second direction opposite to the first direction. In other embodiments, the attachment portions 203 can extend from other parts of the coupler support 200. For example, in one other embodiment, the attachment device 202 can extend from the second transverse elongated portion 222b or the axial elongated portion 221.

In a further aspect of the embodiment, the attachment portions 203 can be flexible members configured to extend at least partially around opposite sides of the recipient's torso, and can include distal ends 205 (shown as a first distal end 205a and a second distal end 205b) configured to releasably engage each other to form a continuous loop around the recipient and secure the coupler support 200 in position. In yet another aspect of this embodiment, the distal ends 205 can include Velcro® to releasably engage each other. In other embodiments, other engagement features can be used to releasably connect the attachment portions 203 together. For example, in other embodiments, a conventional buckle, hook, snap or latch system can be used. In a further embodiment, the distal ends 205 can be tied together in front of the recipient to secure the coupler support 200 in position.

Figure 32 is a partially schematic, side isometric view of the coupler support 200 held in position on a side-lying recipient with an attachment device 206 configured in accordance with another embodiment of the invention. For purposes of clarity, the coupler support 200 is shown schematically in Figure 32 without the couplers 260. In one aspect of that embodiment, the attachment device 206 can include attachment portions 207 (shown as a first attachment portion 207a and a second attachment portion 207b) that do not extend all the way around the recipient. Instead, the attachment portions 207 extend only part of the way around the recipient and are configured to gently squeeze the recipient to hold the coupler support 200 in position. For example, in one embodiment, the attachment portions 207 can include deformable metal members or other materials which a practitioner can easily bend or shape to fit around a particular recipient. In other embodiments, the attachment portions 207 can include other features for attachment to the recipient. For example, in one other embodiment, the attachment portions 207 can include an adhesive backing that releasably adheres to the recipient to secure the coupler support 200 in position.

Both the attachment device 206 and the attachment device 202 (Figure 31) are shown with the coupler support 200 for purposes of illustration only. Accordingly, both of those attachment devices can be used to position other coupler supports having configurations different than that of the coupler support 200. Such coupler supports can include, for example, the coupler supports described below with reference to Figures 33-37.

One feature of embodiments of the attachment devices 202 and 206 described above with reference to Figures 31 and 32, respectively, is that they can secure the coupler support 200 proximate to a coupling area of the recipient even when the coupling area is positioned at an incline. An advantage of that feature is that it enables a practitioner to administer therapy to an inclined recipient without the coupler support 200 falling off the recipient and disrupting the procedure.

Figure 33 is a top isometric view of a coupler support 350 that supports or carries the couplers 260 in accordance with another embodiment of the invention. In one aspect of the embodiment, the coupler support 350 includes a support member 360 and ten engagement members 370 positioned at coupler locations of the support member 360. In another aspect of the embodiment, the coupler support 350 is generally U-shaped and includes two leg portions 361, shown in Figure 33 as a first elongate leg portion 361 a spaced apart from a second elongate leg portion 361b. The leg portions 361 can be positioned to at least partially face each other from opposite sides of a central axis 390 of the support member 360.

Many aspects of the coupler support 350 illustrated in Figure 33 can be at least generally similar in structure and function to corresponding aspects of the coupler support 200 described above with reference to Figures 23, 24 and 25. For example, each of the engagement members 370 can have a columnar or post shape and can be removably received in a downward facing aperture of the coupler 260. In addition, the coupler support 350 can include links 380 extending between the support member 360 and each coupler 260. The links 380 can include electrical cables to transmit electrical signals to the couplers 260 and to the patient or recipient to whom the couplers 260 may be attached. As explained above with reference to the coupler support 200 of Figures 23-25, the different links 380 can have different lengths to allow the corresponding coupler 260 to be coupled to the appropriate site on the recipient. In other embodiments, however, each link 380 can have the same length, as long as each length is sufficient for each coupler 260 to reach the desired site on the recipient.

When the links 380 include electrical cables, each link 380 can enter the support member 360 at a corresponding entry attach port 363. The links 380 can then pass through a cable channel 312 (shown in greater detail in Figures 35A-35C) formed in the support member 360. The links 380 can exit the support member 360 at an exit attachment point 366. As will be described in greater detail below with reference to Figures 35A-35C, in one embodiment, the support member 360 can include an upper portion 310 that includes the cable channel 312, and a lower portion 311 that is molded over the cable channel 312 to embed the links 380 in the support member 360. The links 380 can be bundled together to form a bundled link 351 that can be attached to an electrical connector 352 for coupling to a source of electrical potential.

In a further aspect of the embodiment, the shape of the support member 360 and the positions of the engagement members 370 on the support member 360 can be configured to aid the practitioner in connecting each coupler 260 to the correct corresponding coupling site on the recipient's body. For example, when the coupler support 350 is configured to administer electrical therapy to the recipient's neck or cervical region, the leg portions 361 can extend toward the recipient's head on opposite sides of the body longitudinal axis 271. In that configuration, the coupler support 350 generally, and the leg portions 361 particularly, are spaced apart from corresponding coupling sites on the recipient's neck region so as not to interfere with the operation of attaching the couplers 260 to the recipient. For example, in one embodiment, each of the leg portions 361 can have a length of about 8.0 inches +/- 1.0 inch (measured from where the central axis 390 intersects the leg portions 361), and the leg portions 361 can be spaced apart from each other by about 6.0 inches +/- 1.0 inch. In other embodiments, the leg portions 361 can have other lengths and can be spaced apart from each other by different distances. For example, in another embodiment, each of the leg portions 361 can have a length of about 12.0 inches +/- 1.0 inch, and the leg portions 361 can be spaced apart from each other by about 8.0 inches +/-1.0 inch. In any of those embodiments, each coupler 260 when residing on the support member 360 can be positioned at least proximate to its corresponding coupling site on the recipient's cervical region to aid the practitioner in connecting each coupler to the correct coupling site, as described in more detail below with reference to Figure 34.

Figure 34 is a partially schematic, top isometric view of the coupler support 350 (shown in phantom lines) of Figure 33 positioned near coupling positions on a recipient. For purposes of clarity, the coupler support 350 is shown schematically in Figure 34 without the couplers 260. In one aspect of the embodiment, the central axis 390 of the coupler support 350 is aligned with the body longitudinal axis 271 to position the coupler support 350 proximate to a coupling area 382 on the recipient. The coupling area 382 can be on the recipient's neck and upper back region or cervical region (as shown in Figure 34) or, alternatively, the coupling region 382 can be on the recipient's lower back, head, leg or other body part. When the coupler support 350 is in position on the coupling area 382, the leg portions 361 can flex to conform to the shape of the recipient's cervical region proximate to the coupling area 382. Accordingly, the coupler support 350 can be less likely to be dislodged from the recipient's body and can more accurately align the couplers 260 adjacent to their appropriate coupling positions of the coupling area 382.

The coupling area 382 includes a plurality of sites or coupling positions 381 (shown as 381a-j) at which a corresponding plurality of electrode assemblies 330 (shown as 330a-j) can be attached to the recipient. In one aspect of the embodiment, the electrode assemblies 330 can be at least generally similar in structure and function to the electrode assemblies 230 described above with reference to Figure 24. In other embodiments, other assemblies can be used. For example, in other embodiments, electrode assemblies at least generally similar to those described above with reference to Figures 27-29, or percutaneous hollow needle assemblies generally similar to those described above with reference to Figure 30, can be attached to the recipient at the coupling positions 381. In embodiments for which the assemblies include electrodes the electrode assemblies 330 can be arranged in cathode/anode pairs with five circuits formed by electrode assembly pairs 330a and 330b; 330c and 330d; 330e and 330f; 330g and 330h; and 330i and 330j.

Once the coupler support 350 is in position on the recipient's upper back region, each engagement member 370 (shown as 370a-j) is positioned proximate to its corresponding electrode assembly 330a-j. For example, those engagement members 370 corresponding to electrode assemblies 330 positioned close to the recipient's neck are positioned on distal ends of the leg portions 361. Those engagement members 370 corresponding to electrode assemblies 330 positioned further away from the recipient's neck are positioned on the leg portions 361 proximate to the exit attachment point 366. As a result of the arrangement, many of the couplers 260 (Figure 33) are positioned closer to the one corresponding electrode assembly 330 to which that particular coupler 260 is to be connected than to any other electrode assembly 330. Accordingly, practitioners will be less likely to mix up the couplers 260 or otherwise link the couplers 260 to the incorrect electrode assembly 330.

Figures 35A-C are cross-sectional end views of a series of mold assemblies illustrating a method of manufacturing the support member 360 (Figure 33) or other flexible carrier in accordance with an embodiment of the invention. Although the discussion that follows refers to the support member 360 for purposes of illustration, the methods described can be used in other applications where one or more flexible members, such as an electrical wire or cable, are embedded within a flexible outerbody, such as an elastic carrier.

Referring first to Figure 35A, in one aspect of the embodiment, the upper portion 310 of the support member 360 can be formed using a first mold assembly 324 having a first mold portion 325 and a second mold portion 326. The first mold portion 325 can have a cross-sectional shape 328 that includes the exterior features of the upper portion 310. The second mold portion 326 can have a cross-sectional shape 338 that includes the internal features of the upper portion 310, such as the cable channel 312. In another aspect of the embodiment, the internal features of the upper portion 310 include two lips 313 that extend at least partially inward over the cable channel 312. As explained in greater detail below with reference to Figure 35C, the lips 313 can help to retain a cable harness 358 in the cable channel 312 during the molding process. In other embodiments, the lips 313 can be omitted. A first portion of uncured, low durometer, bio-compatible, thermoplastic elastomeric material 327, such as Santoprene®, or other elastic or nonelastic materials, can be injected into a cavity between the first mold portion 325 and the second mold portion 326 to form the upper portion 310. After the first portion of material 327 has at least partially cured, the first mold portion 325 and the second mold portion 326 can be separated from each other and the upper portion 310 can be removed from the first mold assembly 324. Curing, as used herein, refers broadly to any at least partial hardening of the material 327, and can be achieved in a number of different ways depending on the nature of the material 327. For example, in one aspect of the embodiment the material 327 can cure at room temperature and/or pressure. In another embodiment, the material 327 can cure at an elevated temperature and/or pressure. In yet other embodiments, the material 327 can cure under other conditions.

Referring next to Figure 35B, the upper portion 310 can be positioned in a third mold portion 346 of a second mold assembly 329. In one aspect of the embodiment, the third mold portion 346 has a cross-sectional shape 347 that is at least approximately similar to the cross-sectional shape 328 of the first mold portion 325 (Figure 35A). In another aspect of the embodiment, however, the cross-sectional shape 347 is slightly smaller than the cross-sectional shape 328, causing the upper portion 310 of the elastomeric material 327 to protrude slightly above a surface 348 of the third mold portion 346 when positioned in the third mold portion 346. After the upper portion 310 is positioned in the third mold portion 346, the cable harness 3 58 (which can include, for example, the bundled link 351 and the individual links 380 of Figure 33) can be positioned in the cable channel 312. A fourth mold portion 349 can then be positioned over the third mold portion 346 and pressed against the surface 348 to compress the upper portion 310 of the elastomeric material 327 into the third mold portion 346.

Referring next to Figure 35C, in a further aspect of the embodiment, once the fourth mold portion 349 is mated to the third mold portion 346, a second portion of uncured thermoplastic elastomeric material 327, or other material, can be injected into the second mold assembly 329 to form the lower portion 311 of the support member 360. The compression of the upper portion 310 against the fourth mold portion 349 can help to contain the second portion of material 327 in the cable channel 312 as the second portion of the material 327 is injected and cured. In addition, the lips 313 can help retain the cable harness 358 in the cable channel 312 during this portion of the process. After the second portion of material 327 has at least partially cured, the third and fourth mold portions 346, 349 can be separated and the support member 360 can be lifted from the second mold assembly 329.

In other embodiments, other methods can be used to manufacture the support member 360 (Figure 33). For example, in one embodiment, the upper portion 310 and the lower portion 311 of the support member 360 can be formed using a single mold assembly. In one aspect of the embodiment, the upper portion 310 is made with the first mold assembly 324 as explained above with reference to Figure 35A. After the upper portion has at least partially cured, the second mold portion 326 is separated from the first mold portion 325 and the second mold portion 326 is extracted from the upper portion 310 of the support member 360. The fourth mold portion 349 can then be positioned against the first mold portion 325, and the second portion of the material 327 can be injected into the cable channel 312 to form the lower portion 311 as explained above with reference to Figure 35C. In another aspect of the embodiment, compression of the upper portion 310 to prevent leakage of the second portion of the material 327 can be provided in a number of ways. One such way is to provide the fourth mold portion 349 with protruding portions that extend downward and compress the upper portion 310 when the fourth mold portion 349 is mated to the first mold portion 325. Another way is to provide a shim or spacer on the upper surface of the first mold portion 325 that can be removed after the upper portion 310 has been formed. Removal of the spacer will cause the upper portion 310 to protrude slightly above the first mold portion 325 (as shown in Figure 35B with reference to the third mold portion 346). As a result, mating the fourth mold portion 349 to the first mold portion 325 will compress of the upper portion 310 and provide a seal.

In yet another aspect of the embodiment, the upper portion 310 and the lower portion 311 can be formed using the method outlined above with low durometer, bio-compatible, thermoplastic elastomeric materials. For example, in one embodiment, the upper portion 310 and the lower portion 311 can be formed using elastic materials having durometers from about 55 Shore A to about 87 Shore A, or from about 40 Shore D to about 50 Shore D. In another embodiment, the upper portion 310 and the lower portion 311 can be formed using Santoprene® having a durometer of about 73 Shore A. In further embodiments, still other materials having other hardness/softness characteristics can be used.

One feature of embodiments of the manufacturing method described above with reference to Figures 35A-C is that the cable harness 358 is captured within the cable channel 312 during the molding process. One advantage of that feature is that it prevents the cable harness 358 from stretching, flexing or "surfacing" as the thermoplastic elastomer is injected into the mold assembly. The term surfacing describes the situation where the cable harness 358 or other flexible member moves in a mold assembly to a position at or near a mold surface. Such surfacing can leave portions of the cable harness 358 exposed or relatively unprotected and result in premature wear or failure of one or more of the individual links 380. A further advantage of the feature is that the cable harness 358 is embedded within the upper portion 310 in a protective manner that organizes the individual links 380 but still allows them to flexibly conform to uneven surfaces upon which the support member 360 is positioned.

Figure 36 is a partially schematic, side isometric view of a coupler support 365 configured in accordance with an embodiment of the invention and positioned on a recipient having an upright or semi-upright posture. In one aspect of the embodiment, the coupler support 365 includes a support member 366 having elongate leg portions 364 that are at least generally similar in structure and function to the elongate leg portions 361 of the support member 360 described above with reference to Figures 33 and 34. In another aspect of the embodiment, however, the support member 366 includes a deformable member 367 that allows the leg portions 364 to be bent and/or shaped to conform to shoulders of the recipient. Forming the leg portions 364 in that manner allows the coupler support 365 to engage the shoulders of the recipient proximate to a coupling area, such as the coupling area 382 described above with reference to Figure 34. The feature allows a practitioner to use the coupler support 365 in, for example, the manner described above with reference to Figure 34, even when the recipient is in an upright, or semi-upright, seated or standing position.

Figure 37 is a cross-sectional end view of the support member 366 of Figure 36 taken substantially along line 37-37 in Figure 36. In one aspect of the embodiment, the support member 366 includes an upper portion 410, a lower portion 411, and a cable harness 458 positioned in a cable channel 412. Those features of the support member 366 can be at least generally similar in structure, function and manufacture to the corresponding features of the support member 360 described above with reference to Figures 33 and 35A-C. In another aspect of the embodiment, the deformable member 367 of the support member 366 is positioned in the cable channel 412 with the cable harness 458. In other embodiments, the deformable member 367 can be embedded in other portions of the support member 366. For example, in one other embodiment, the deformable member 367 can be molded into the upper portion 410, as shown by the phantom lines in Figure 37.

In yet another aspect of the embodiment, the deformable member 367 can include a deformable wire or other deformable member having an elastic jacket 459 with a durometer that is greater than the durometer of the upper portion 410 and the lower portion 411. For example, in one embodiment, the upper portion 410 and the lower portion 411 can have durometers of about 73 Shore A, and the jacket 459 can have a durometer of about 87 Shore A. In other embodiments, other materials having other hardness/softness characteristics can be used for the jacket 459, the upper portion 410, and the lower portion 411. Providing the jacket 459 with a durometer greater than the surrounding material of the support member 366 can prevent the deformable member 367 from breaking through or otherwise damaging the support member 366 when the deformable member 367 is flexed or bent into a particular shape to fit on a recipient. In further embodiments, the individual links 380 and/or the cable harness 358 can also be provided with elastic jackets of higher durometers than the surrounding material of the support member 366.

In a further aspect of the embodiment, the deformable member 367 can include a ductile metal such as copper, steel or aluminum. For example, in one embodiment, the deformable member 367 can include an annealed 302 or 304 stainless steel rod having a diameter of from about .040 inch to about .064 inch. In other embodiments, the deformable member 367 can include other materials, such as nonmetallic plastic materials. In yet another aspect of the embodiment, the jacket 459 can have an outer diameter of about .10 inch to about .20 inch. For example, in one embodiment, the jacket 459 can have an outer diameter of .15 inch. In other embodiments, the jacket can have other diameters or can be omitted. Although the deformable member 367 of the illustrated embodiment is shown as a wire, such as a copper, steel or aluminum wire or other element having a circular cross section, in other embodiments, other deformable materials can be used. For example, in another embodiment, the deformable member 367 can include a flat or rectangular piece of ductile metal, such as annealed steel or aluminum. In yet other embodiments, the deformable member 367 can include other deformable materials.

Figure 38 is a partially schematic, top isometric view of the coupler support 350 (shown in phantom lines) of Figure 33 positioned proximate to coupling area 384 on a lower back region of a recipient. For purposes of clarity, the coupler support 350 is shown schematically in Figure 38 without the couplers 260 (Figure 33). The coupling area 384 includes a plurality of sites or coupling positions 383 (shown as 383a-j) at which a corresponding plurality of the electrode assemblies 330 (shown as 330a-j) can be attached to the recipient. Once the coupler support 350 is in position on the recipient's lower back region, each engagement member 370 (shown as 370a-j) is positioned proximate to its corresponding electrode assembly 330a-j. As a result, many of the couplers 260 are positioned closer to the one corresponding electrode assembly 330 to which that particular coupler 260 is to be connected than to any other electrode assembly 330. Accordingly, practitioners will be less likely to mix up the couplers 260 or otherwise link the couplers 260 to the incorrect electrode assembly 330.

From the foregoing, it will be appreciated that, although specific embodiments of the invention have been described herein for purposes of illustration, various modifications may be made without deviating from the spirit and scope of the invention. For example, the couplers can be connected directly to the recipient rather than being connected to an intermediate device such as an electrode (i.e., the electrode can be integrated with the coupler). The coupler locations of the support member can include posts or columns, apertures, or any other feature that removably carries the couplers. Accordingly, the invention is not limited except as by the appended claims.

## Claims

1. An apparatus for supporting couplers for removable coupling to a recipient during at least one of therapy administration and recipient monitoring, the apparatus comprising a support member configured to rest on a body of the recipient, the support member having a first coupler support portion configured to be positioned proximate to a first coupling position of the body of the recipient, the support member further having a second coupler support portion configured to be positioned proximate to a second coupling position of the body of the recipient, the first coupler support portion being configured to removably carry a first coupler, the second coupler support portion being configured to removably carry a second coupler, and wherein the support member includes a deformable member positioned at least proximate to the first and second coupler support portions, the deformable member being bendable from a first shape to a second shape and configured to at least generally maintain its shape after being bent.

2. The apparatus of claim 1, wherein the first coupler support portion is configured to accommodate movement of the first coupler between a first carried position with the first coupler carried by the first coupler support portion and a first coupled position with the first coupler operatively coupled to the recipient at the first coupling position, and wherein the second coupler support portion is configured to accommodate movement of the second coupler between a second carried position with the second coupler carried by the second coupler support portion and a second coupled position with the second coupler operatively coupled to the recipient at the second coupling position.

3. The apparatus of claim 1, wherein the first coupler support portion includes an engagement member configured to releasably support the first coupler, and wherein the apparatus further comprises a flexible cable configured to be connected between the first coupler and the support member, the cable remaining connected between the first coupler and the support member when the first coupler is moved from the first carried position to the first coupled position.

4. The apparatus of claim 1, wherein the support member includes a first flexible material having a first durometer, and wherein the deformable member is encased in a second flexible material having a second durometer greater than the first durometer.

5. The apparatus of claim 1, wherein the first and second coupler support portions are two of a larger plurality of coupler support portions, wherein each of the larger plurality of coupler support portions is configured to be positioned proximate to a corresponding one of a larger plurality of coupling positions including the first and second coupling positions, and further wherein an outline of the coupling positions defines a first shape and an outline of the coupler support portions defines a corresponding second shape at least generally similar to the first shape.

6. The apparatus of claim 1, wherein the support member includes first and second elongate leg portions at least generally arranged in a U-shape, and wherein the deformable member extends at least proximate to the first and second elongate leg portions.

7. An apparatus for supporting a plurality of percutaneous probe couplers in position for removable coupling to a recipient, the apparatus comprising:
a support member configured to rest on a body of the recipient, the support member including a deformable member bendable from a first shape to a second shape and configured to at least generally maintain its shape after being bent;
a first engagement member depending from the support member and configured to be positioned proximate to a first coupling position on the body;
a first coupler removably engaged with the first engagement member;
a first electrical cable attached between the first coupler and the support member;
a second engagement member depending from the support member and configured to be positioned proximate to a second coupling position on the body of the recipient, the first engagement member configured to be positioned closer than the second engagement member to the first coupling position and the second engagement member configured to be positioned closer than the first engagement member to the second coupling position;
a second coupler removably engaged with the second engagement member; and
a second electrical cable attached between the second coupler and the support member.

8. The apparatus of claim 7, wherein the first electrical cable is attached to the support member at a first attachment location and the second elocttical cable is attached to the support member at a second attachment location, and wherein the first and second electrical cables are bundled together within the support member and exit the support member adjacent to each other at a third attachment location.

9. An apparatus for administering therapy to a recipient, monitoring the recipient, or administering therapy and monitoring the recipient, the apparatus comprising:
a support member configured to rest on a body of the recipient, the support member including a deformable member configured to facilitate shaping of the support member to fit a contour of a body of the recipient, the support member having a first coupler support portion configured to be positioned proximate to a first coupling position of the body of the recipient, the support member further having a second coupler support portion configured to be positioned proximate to a second coupling position of the body of the recipient;
a first coupler configured to be operatively coupled to the body at a first coupling position and removably supported at the first coupler support portion;
a second coupler configured to be operatively coupled to the body at a second coupling position and removably supported at the second coupler support portion;
a recipient care unit configured to deliver therapy to the recipient, monitor a condition of the recipient, or delivery therapy and monitor a condition of the recipient;
a first link between the care unit and the first coupler; and
a second link between the care unit and the second coupler.

10. An apparatus for supporting at least first and second couplers for removable coupling to at least first and second coupling positions on a body of a recipient during at least one of therapy administration and recipient monitoring, the apparatus comprising:
a support member configured to be positioned on the recipient, the support member having a first coupler support portion configured to be positioned proximate to the first coupling position of the body of the recipient and removably carry the first coupler, the support member further having a second coupler support portion configured to be positioned proximate to the second coupling position of the body of the recipient and removably carry the second coupler; and
an attachment device depending from the support member and configured to extend at least partially around the body of the recipient to releasably hold the support member in position on the recipient at least proximate to the first and second coupling positions.

11. The apparatus of claim 10, wherein the first coupler support portion is configured to accommodate movement of the first coupler between a first carried position with the first coupler carried by the first coupler support portion and a first coupled position with the first coupler operatively coupled to the recipient at the first coupling position, wherein the second coupler support portion is configured to accommodate movement of the second coupler between a second carried position with the second coupler carried by the second coupler support portion and a second coupled position with the second coupler operatively coupled to the recipient at the second coupling position, and wherein the attachment device includes a first portion extending from the support member in a first direction and a second portion extending from the support member in a second direction opposite to the first direction.

12. The apparatus of claim 10, wherein the attachment device includes a first flexible strap portion extending from the support member in a first direction and a second flexible strap portion extending from the support member in a second direction opposite to the first direction, wherein each of the first and second strap portions include distal ends configured to releasably engage each other to form a continuous strap around the recipient.

13. A method for at least one of administering therapy to a recipient and monitoring the recipient, the method comprising:
removably positioning at least one coupler on a conformable support member; bending at least a portion of the support member to make the support member at least generally conform to a contour of a body of the recipient;
positioning the support member on the body of the recipient;
removing the at least one coupler from the support member; and
removably coupling the at least one coupler to a coupling position on the body of the recipient at least proximate to the positioned support member.

14. The method of claim 13, wherein removably positioning the at least one coupler on the conformable support member includes removably positioning a first coupler on a first elongate portion of the support member, and wherein the method further comprises removably positioning a second coupler on a second elongate portion of the support member spaced apart from the first elongate portion.

15. The method of claim 13, wherein removably positioning the at least one coupler on the conformable support member includes removably positioning a first coupler on a first elongate portion of the support member, wherein the method further comprises removably positioning a second coupler on a second elongate portion of the support member spaced apart from the first elongate portion, and wherein bending the at least a portion of the support member includes bending the first and second elongate portions of the support member to make them at least generally conform to a shoulder region of the recipient

16. The method of claim 13, wherein removably positioning the at least one coupler on the conformable support member includes removably positioning a first coupler on a first engagement member depending from the support member and removably positioning a second coupler on a second engagement member depending from the support member, the second engagement member being spaced apart from the first engagement member, and wherein removably coupling the at least one coupler to the coupling position on the body of the recipient includes removably coupling the first coupler to a first coupling position at least proximate to the first engagement member and removably coupling the second coupler to a second coupling position at least proximate to the second engagement member.

17. A method for coupling at least one of therapy equipment and monitoring equipment to a recipient, the method comprising:
positioning first and second legs of a U-shaped support member against a body of a recipient proximate to a coupling area of the body and spaced apart from first and second coupling positions in the coupling area;
supporting a first coupler relative to the body at a first coupler support portion of the first leg of the support member proximate to the first coupling position;
supporting a second coupler relative to the body at a second coupler support portion of the second leg of the support member proximate to the second coupling position;
removing the first coupler from the first coupler support portion of the first leg of the support member and coupling the first coupler to the body at the first coupling position; and
removing the second coupler from the second coupler support portion of the second leg of the support member and coupling the second coupler to the body at the second coupling position.

18. The method of claim 17, wherein positioning the first and second legs of the support member includes positioning a first elongate portion of the support member at least generally on one side of a spine of the recipient, and positioning a second elongate portion at least generally on the other side of the spine of the recipient

19. A method for administering percutaneous electrical therapy to a recipient, the method comprising:
positioning first and second legs of a U-shaped flexible support member against a body of the recipient proximate to a coupling area having first and second coupling positions;
supporting a first coupler relative to the body at a first coupler support portion of the first leg of the support member proximate to the first coupling position;
supporting a second coupler relative to the body at a second coupler support portion of the second leg of the support member proximate to the second coupling position, the first coupler support portion being positioned closer than the second coupler support portion to the first coupling position;
removing the first coupler from the first coupler support portion of the first leg and electrically coupling the first coupler to a first percutaneous probe positioned in the body at the first coupling position;
removing the second coupler from the second coupler support portion of the second leg and electrically coupling the second coupler to a second percutaneous probe in the body at the second coupling position; and
electrically coupling the first and second couplers to a source of electrical potential.
